(19) Europäisches Patentamt
European Patent Office
Office européen des brevets

(11) EP 2 246 320 A1

(12) **EUROPÄISCHE PATENTANMELDUNG**

(43) Veröffentlichungstag:
**03.11.2010 Patentblatt 2010/44**

(51) Int Cl.:
***C07C 209/36*** *(2006.01)* ***C07C 211/46*** *(2006.01)*

(21) Anmeldenummer: **10004248.0**

(22) Anmeldetag: **21.04.2010**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA ME RS**

(30) Priorität: **29.04.2009 DE 102009019436**

(71) Anmelder: **Bayer MaterialScience AG**
**51368 Leverkusen (DE)**

(72) Erfinder:
- **Sommer, Knut, Dr.**
  **47804 Krefeld (DE)**
- **Wilke, Karl-Heinz**
  **47447 Moers (DE)**
- **Lehner, Peter, Dr.**
  **45481 Mülheim/Ruhr (DE)**
- **Gehlen, Franz-Ulrich**
  **47839 Krefeld (DE)**
- **Mleczko, Leslaw, Prof.Dr.**
  **41542 Dormagen (DE)**
- **Schubert, Stephan, Dr.**
  **Baytown, 77520-8727 (US)**
- **Bellinghausen, Rainer , Dr.**
  **51519 Odenthal (DE)**
- **Hizaler Hoffmann, Evin**
  **50733 Köln (DE)**

(54) **Verfahren zur Herstellung von aromatischen Aminen**

(57) Die Erfindung betrifft ein Verfahren zur Hydrierung von Nitroaromaten zu aromatischen Aminen in der Gasphase mit Wasserstoff an in stationären oder quasistationären Betten angeordneten Katalysatoren in einem Reaktor, bei dem der in dem Reaktor befindliche Katalysator kontinuierlich oder in periodischen Abständen zumindest teilweise ausgetauscht wird, wobei innerhalb von 20 Tagen mindestens 10 % des Katalysators ausgetauscht werden.

EP 2 246 320 A1

## Beschreibung

Die Erfindung betrifft ein Verfahren zur Hydrierung von Nitroaromaten zu aromatischen Aminen in der Gasphase mit Wasserstoff an in stationären oder quasi-stationären Betten angeordneten Katalysatoren in einem Reaktor, bei dem der in dem Reaktor befindliche Katalysator kontinuierlich oder in periodischen Abständen zumindest teilweise ausgetauscht wird, wobei innerhalb von 20 Tagen mindestens 10 % des Katalysators ausgetauscht werden.

Aromatische Amine sind wichtige Zwischenprodukte, welche preiswert und in großen Mengen zur Verfugung stehen müssen. Insbesondere Anilin hat große Bedeutung als Zwischenprodukt in der Herstellung von Di- und Polyisocyanaten der Diphenylmethanreihe (im Folgenden summarisch als MDI bezeichnet):

MDI wird nach dem Stand der Technik aus den korrespondierenden Di- und Polyaminen gewonnen, in der Regel durch Phosgenierung. Die Di- und Polyamine der Diphenylmethanreihe (im Folgenden summarisch als MDA bezeichnet) werden durch Reaktion von Anilin mit Formaldehyd hergestellt. Das Anilin wiederum wird großtechnisch meist durch Hydrierung von Nitrobenzol produziert. Letzteres erhält man durch Nitrierung von Benzol, sodass die gesamte Prozesskette vereinfacht wie folgt dargestellt werden kann:

| | | | | | | | | |
|---|---|---|---|---|---|---|---|---|
| | *Nitrie-rung* | | *Hydrie-rung* | | *Kopplung mit Formaldehyd* | | *Phosge-nierung* | |
| Benzol | → | Nitrobenzol | → | Anilin | → | MDA | → | MDI |

Handelsübliches Benzol kann je nach Quelle mehr oder weniger stark verunreinigt sein. Typische Verunreinigungen sind andere Aromaten, insbesondere Toluol und Xylol, die in Benzol gängiger Reinheit bis zu jeweils 0,05 Massen-% enthalten sein können. Andere für Benzol typische Verunreinigungen sind nichtaromatische organische Verbindungen, die in Summe bis zu 0,07 Massen-% ausmachen können. Insbesondere sind hier Cyclohexan (bis zu 0,03 Massen-%) und Methlycyclohexan (bis zu 0,02 Massen-%) zu nennen. Die vorstehend beschriebenen Verunreinigungen stören die nachfolgenden Schritte in der MDI-Prozesskette in den genannten Konzentrationen entweder überhaupt nicht oder nur geringfügig, beispielsweise durch eine minimale Erschwerung der Abwasser- und Abluftaufbereitung im Nitrobenzolverfahren durch nichtaromatische Organika im Benzol. Eine aufwändige Reinigung des Benzols für den Einsatz in der MDI-Prozesskette wäre daher unverhältnismäßig und kann unterbleiben. Aufgrund der großen industriellen Bedeutung der Di- und Polyisocyanate der Diphenylmethanreihe müssen für die Hydrierung von Nitrobenzol zu Anilin Anlagen mit sehr großen Kapazitäten gebaut werden.

Die Hydrierung von Nitroaromaten ist eine stark exotherme Reaktion. So werden beispielsweise bei 200 °C bei der Hydrierung von Nitroxylol zu Xylidin ca. 488 kJ $mol^{-1}$ und bei der Hydrierung von Nitrobenzol zu Anilin ca. 544 kJ $mol^{-1}$ freigesetzt. Die Abführung und Verwendung der Reaktionswärme ist sowohl aus ökologischer als auch aus wirtschaftlicher Sicht ein wichtiger Punkt bei der Durchführung von Verfahren zur Hydrierung von Nitroaromaten.

So wird in einer etablierten Verfahrensweise der Katalysator als fluidisiertes, thermostabilisiertes Bett betrieben (DE-B-1 114 820). Der effektiven Wärmeabfuhr dieser Verfahrensweise stehen Probleme durch uneinheitliche Verweilzeitverteilung (Nitrobenzoldurchbruch) und Katalysatorabrieb gegenüber. Das Patent DE-B-1 133 394 lehrt zusätzlich noch die Fahrweise unter Druck, wodurch die Lebensdauer der Katalysatoren verlängert werden soll. Ein neuerer Ansatz mit fluidisiertem Katalysatorbett (WO 2008/034770 A1) beschreibt einen Reaktor mit Einbauten, welche die Wirbelschicht in eine Mehrzahl von horizontal sowie in eine Mehrzahl von vertikal angeordneten Zellen austeilt, wodurch der Stoffübergang und damit der Umsatz verbessert werden soll, was aber natürlich die Reaktorkonstruktion erheblich kompliziert.

Enge Verweilzeitverteilungen und geringer Katalysatorabrieb sind grundsätzlich in Reaktoren realisierbar, in denen die Katalysatorschüttung während des Hydrierprozesses stationär ist, was im Folgenden als Festbett bezeichnet wird.

Zwei Festbett-Reaktortypen werden häufig eingesetzt: Zum einen Rohrbündelreaktoren, welche zur Thermostatisierung der Katalysatorbetten einen Kühlkreislauf besitzen (sog. "*isotherme Fahrweise* ", siehe bspw. DE-OS 2 201 528). Daneben gibt es Reaktorkonstruktionen, welche nur Katalysatorschüttungen auf bzw. zwischen einfachen Auflagerosten und/oder Metallsieben enthalten und kein System zur Wärmehaushaltung im Reaktor besitzen, d. h., dass Maßnahmen zur Thermostatisierung der Katalysatorbetten, etwa durch Wärmeträgeröl, völlig entfallen. Die Reaktionsenthalpie spiegelt sich bei diesem Reaktortyp in der Temperaturdifferenz zwischen Edukt- und Produktgasstrom (bis auf ggf. unvermeidbare Wärmeverluste) quantitativ wieder (sog. "*adiabate Fahrweise*"). Im Folgenden wird der Stand der Technik mit Beispielen aus beiden Fahrweisen dargelegt:

GB 1.452.466 befasst sich mit einem Verfahren zur Nitrobenzolhydrierung, bei dem ein adiabater Reaktor einem isothermen Reaktor nachgeschaltet ist. Dabei wird der größte Teil des Nitrobenzols in einem thermostatisierten Rohrbündelreaktor umgesetzt; lediglich die Hydrierung des Restgehaltes an Nitrobenzol erfolgt bei relativ geringem

Wasserstoffüberschuss (kleiner 30 : 1) in einem adiabaten Reaktor.

DE-OS 1 809 711 befasst sich mit dem gleichmäßigen Einbringen von flüssigen Nitroverbindungen in einen heißen Gasstrom durch Verdüsen, bevorzugt an verengten Stellen unmittelbar vor dem Reaktor. Auf den Aufbau des Reaktors wird in DE-OS 1 809 711 nicht eingegangen.

In DE-OS 36 36 984 wird ein Verfahren zur gekoppelten Produktion von Nitro- und Dinitroaromaten aus den entsprechenden Kohlenwasserstoffen durch Nitrierung und deren nachfolgende Hydrierung beschrieben. Die Hydrierung erfolgt in der Gasphase bei Temperaturen von zwischen 176 °C und 343,5 °C. Es wird eine Apparatur zur Gasphasenhydrierung beschrieben, die im Wesentlichen aus zwei hintereinander geschalteten Reaktoren mit Zwischenkühlung und Eduktzwischeneinspeisung besteht, auf deren Größe und Aufbau nicht eingegangen wird.

In den genannten Veröffentlichungen werden bei niedrigen Belastungen und auf niedrigem Temperaturniveau betriebene Kupfer-Katalysatoren eingesetzt, woraus geringe Raum-Zeit-Ausbeuten resultieren.

Neben den erwähnten Kupfer-Katalysatoren sind zahlreiche andere Kontakte für die Gasphasenhydrierung von Nitroaromaten beschrieben. Sie wurden in vielen Publikationen beschrieben und umfassen als hydrieraktive Elemente Pd, Pt, Ru, Fe, Co, Ni, Mn, Re, Cr, Mo, V, Pb, Ti, Sn, Dy, Zn, Cd, Ba, Cu, Ag, Au, und deren Verbindungen, zum Teil als Oxide, Sulfide oder Selenide und auch in Form einer Raney-Legierung sowie auf Trägem, wie $Al_2O_3$, $Fe_2O_3/Al_2O_3$, $SiO_2$, Silikaten, Kohle, $TiO_2$, $Cr_2O_3$.

In DE-A-2 244 401 und DE-A-2 849 002 werden Palladium-Katalysatoren auf Aluminiumoxid-Trägern beschrieben, die als stationäre Katalysatorschüttungen in Wärmeaustauscherrohren unter Normaldruck bei Belastungen von weniger als 1 $g_{Nitroaromat}/[ml_{Katalysator} \cdot h]$ mit geringen Wasserstoff-Nitrobenzol-Verhältnissen betrieben werden.

In DE 4 039 026 A1 werden Palladium-Katalysatoren auf graphitischen Trägern beschrieben, die unter ähnlichen Bedingungen wie die Palladium-Katalysatoren auf Aluminiumoxid betrieben werden. Bei all diesen Verfahrensvarianten muss die große anfallende Reaktionswärme einem technischen Reaktor über ein Wärmeträgersystem entzogen werden.

DE 196 51 688 A1 beschreibt ein Verfahren zur Herstellung von aromatischen Aminen, bei dem die spezifische Belastung des Katalysators mit aromatischer Nitroverbindung kontinuierlich oder stufenweise auf Werte bis zu 5,0 $kg_{Nitroverbindung} / (l_{Katalysator} \cdot h)$ gesteigert wird, wodurch hohe Raum-Zeit-Ausbeuten erzielt werden. In besonderen Ausführungsformen werden die Katalysatorschüttungen durch inerte Füllkörper verdünnt und weisen ggf. Aktivitätsgradienten auf. Die Anmeldung beschreibt Hydrierungen in thermostatisierten Reaktoren bei verhältnismäßig geringen Wasserstoffüberschüssen. Die positiven Effekte der kontinuierlichen oder stufenweisen Laststeigerung und der Verdünnung der Katalysatorschüttung sind jedoch nicht Folge des speziellen Reaktortyps oder des Wasserstoffüberschusses. Daher ist die Lehre aus DE 196 51 688 A1 auch auf eine adiabate Prozessführung mit hohen Wasserstoffüberschüssen anwendbar.

Mit einem unter rein adiabaten Bedingungen durchgeführten Verfahren befassen sich die Patente EP 0 696 573 B1, EP 0 696 574 B1, EP 0 748 789 B1, EP 0 748 790 B1 sowie EP 1 882 681 A1. EP 0696574 B1 beschreibt den Prozess zur Herstellung von aromatischen Aminen, in welchem der Katalysator unter adiabaten Bedingungen mit einem Gasgemisch bestehend aus Nitroaromaten und Wasserstoff angeströmt wird, in ganz allgemeiner Weise. In den Verfahren gemäß der Patente EP 0 696 573 B1, EP 0 748 789 B1, EP 0 748 790 B1 sowie EP 1 882 681 A1 werden durch Änderung verschiedener Parameter jeweils bestimmte Vorteile erzielt:

EP 0 696 573 B1 beschreibt den Vorteil besonders hoher Selektivitäten, wenn der umzusetzende Nitroaromat außer mit Wasserstoff auch mit einem Vielfachen des bei der Reaktion entstehenden aromatischen Amins und einem Vielfachen an Wasser über den Katalysator geleitet wird. Bei dieser Fahrweise sind in jedem Katalysatorvolumen pro Mol Nitrogruppe mindestens 2 Mol Aminogruppen und 4 Mol Wasser vorhanden. Die beschriebenen Katalysatoren sind die gleichen wie in EP 0 696 574 B1. Nachteilig bei dieser Verfahrensweise ist, dass große Mengen für die eigentliche Reaktion im Prinzip entbehrlicher Verbindungen, nämlich Wasser und Amin, ständig im Kreis geführt werden müssen. Insbesondere die ständige Rezyklisierung von mindestens 2 Äquivalenten des entstehenden Amins, also des wertvollen Produktes des Verfahrens, ist von großem Nachteil, da das hergestellte Amin dadurch mehrfach thermisch stark belastet wird.

Die Patente EP 0 748 789 B1 und EP 0 748 790 B1 beschreiben die Erzielung von Vorteilen lediglich durch Verwendung spezieller Katalysatorsysteme:

- Palladium-Katalysatoren auf Graphit oder graphithaltigem Koks mit einem Palladiumgehalt > 1,5 und ≤ 7 Massen-% (EP 0 748 789 B1). Der Vorteil liegt in den außerordentlich langen Standzeiten im Vergleich zu allen früher beschriebenen Katalysatoren. Nachteil dieses Verfahrens sind die mit der hohen Palladiumkonzentration unweigerlich verbundenen immens hohen Kosten des Katalysators. Das Patent geht nicht darauf ein, ob die hohen Katalysatorkosten bei den für eine großtechnische Anwendung erforderlichen großen Mengen an Palladium noch durch die langen Standzeiten kompensiert werden können.
- Palladium-Blei-Katalysatoren auf Graphit oder graphithaltigem Koks mit einem Palladiumgehalt von 0,001 bis 7

Massen-% (EP 0 748 790 B1). Der Vorteil liegt in den höheren Selektivitäten im Vergleich zu analogen Katalysatoren ohne Bleizusatz. Bei allen im Patent beschriebenen Beispielen kamen Katalysatoren mit 2 Massen-% Palladium zum Einsatz, so dass der Nachteil hoher Katalysatorkosten auch in diesem Fall voll zum Tragen kommt.

Die Patentanmeldung EP 1 882 681 A1 beschreibt Vorteile, die dadurch erzielt werden, dass der Eduktgasstrom zu Beginn der Hydrierung bereits signifikante Mengen an Wasser, jedoch das gebildete aromatische Amin höchstens in geringen, aus dem Kreisgasstrom stammenden Mengen enthält. Hierdurch werden Standzeitverbesserungen erreicht. Außerdem wird eine Selektivitätsverbesserung durch Einspeisung von Stickstoff gelehrt. Auch diese Fahrweise weist noch Nachteile auf: So muss die Produktion in regelmäßigen Abständen zur Regenerierung des Katalysators unterbrochen werden. Neben dem damit unweigerlich verbundenen Produktionsausfall ist ein weiterer und noch viel schwerwiegender Nachteil darin zu sehen, dass jeder neue Produktionszyklus auf einem niedrigen Niveau der Selektivität beginnt. Selektivitätswerte > 99 % werden nur bei Einspeisung erheblicher Mengen Stickstoffs bereits nach kurzer Zeit erreicht (EP 1 882 681 A1, S. 10, Absatz [0081]), was hohe Kosten verursacht. Ein völliger Verzicht auf Stickstoff erschwert die Aufarbeitung, weil die Qualität des aus dem Prozess kommenden rohen Amins unter diesen Bedingungen zyklisch großen Schwankungen unterworfen ist.

Mit der Problematik, *über die gesamte Laufzeit eines Produktionszyklus* eine möglichst *nur sehr geringen Schwankungen* unterworfene Selektivität auf möglichst *hohem Niveau* zu erzielen, befassen sich die bisher genannten Veröffentlichungen entweder gar nicht oder sie bieten lediglich teure und damit unwirtschaftliche Lösungen an, wie die in EP 1 882 681 A1 gelehrte Einspeisung sehr großer Mengen an Stickstoff zu Beginn der Hydrierung.

Eine generelle Verbesserung der Selektivität und Verlängerung der Katalysatorstandzeit kann prinzipiell durch die Verwendung von in flächigen Schichten angeordneten Katalysatorbetten, die senkrecht mit Eduktgas angeströmt werden (sog. "*Radialreaktoren* "), erzielt werden, wie DE 42 07 905 A1 lehrt (S. 6, Z. 61 ff.). Diese Anmeldung beschreibt thermostatisierte Reaktoren. Natürlich kann auch ein adiabat betriebenes Festbettverfahren zur Hydrierung von Nitroaromaten (EP 0 696 574 B1 und Auswahlpatente) in Radialreaktoren durchgeführt werden. Jedoch verhindert eine solche Fahrweise alleine nicht die großen zyklischen Selektivitätsschwankungen.

Signifikante zyklische Selektivitätsschwankungen stellen immer dann ein prinzipielles Problem dar, wenn sich die Aktivität (und damit in der Regel auch die Selektivität) eines Katalysators zu Beginn eines Produktionszyklus sehr stark von der am Ende des Produktionszyklus unterscheidet. Unter "Akti-vität" wird im Rahmen dieser Erfindung die Fähigkeit des Katalysators verstanden, den Nitroaromaten möglichst lange und möglichst vollständig umzusetzen. Zu Beginn eines Produktionszyklus hat der Katalysator seine höchste Aktivität und geringste Selektivität. Im Laufe eines Produktionszyklus nimmt dann die Aktivität, bspw. infolge langsamer Verkokung und/oder Sinterung der katalytisch aktiven Metalle, ab und die Selektivität zu. Wenn der Umsatz auf nicht mehr akzeptable Werte fällt, wird die Produktion unterbrochen und der Katalysator regeneriert. Im nächsten Produktionszyklus liegt der Katalysator dann zunächst in einem Zustand vor, der dem von Frischkatalysator gleicht oder zumindest ähnelt, weist also eine hohe Aktivität und geringe Selektivität auf. Der Katalysator durchläuft also in jedem Produktionszyklus einen großen Alterungsprozess. Die Abhängigkeit der Selektivität des Katalysators in einem gegebenen inkrementellen Volumen des Katalysatorbetts von der Laufzeit ist daher sehr groß, und die zu einem gegebenen Zeitpunkt gemessene momentane Selektivität kann sich deutlich von der über einen ganzen Produktionszyklus gemittelten Selektivität unterscheiden. Aus diesen Ausführungen folgt, dass sich das Problem der großen Selektivitätsschwankungen schon dadurch vermindern lässt, dass man nach Beendigung eines Produktionszyklus bewusst "weniger gut" (also z. B. für kürzere Zeit und/oder bei geringerer Temperatur als es im Sinne maximaler Aktivität optimal wäre) regeneriert. Dies hat zur Folge, dass der zu Beginn der Hydrierung im nächsten Produktionszyklus im FestbettReaktor vorliegende Katalysator sich anders verhält als ein Frischkatalysator desselben Katalysatorsystems, sodass von Anfang an bessere Selektivitäten erzielt werden können als mit vollständig (im Sinne maximaler Aktivität) regeneriertem Katalysator, allerdings zu Lasten der Standzeit. Das Auffinden solcher Regenerierbedingungen, die einen akzeptablen Kompromiss zwischen erhöhter Selektivität und verminderter Standzeit ermöglichen, ist jedoch aufwändig.

Unterbrechungen des Produktionsprozesses infolge Katalysatorregeneration können prinzipiell vermieden und damit auch das Problem großer zyklischer Selektivitätsschwankungen zumindest etwas vermindert werden, wenn laufend bzw. periodisch verbrauchter Katalysator entnommen und frischer oder regenerierter Katalysator zugeführt wird. Dies ist in der Hydrierung aromatischer Nitroverbindungen zwar prinzipiell mit einem Wirbelschichtverfahren möglich, wie die bereits erwähnte Patentschrift DE-B-1 114 820 (Spalte 2, Z. 31 bis 35) und auch CN-A-101 016 247 lehren, löst jedoch nicht die oben genannten grundsätzlichen Probleme dieser Prozessführung.

Darüber hinaus gibt es Reaktoren, die einen Austausch von Katalysator ohne Unterbrechung des Produktionsprozesses gestatten und bei denen der Katalysator nicht in wirbelnde Bewegung versetzt wird, sondern aufgrund der Schwerkraft durch geeignete Schleusensysteme "fließt" (sog. "*Wanderbettreaktoren* "). Bei diesem Reaktortyp kann die Katalysatorschüttung während der Reaktion bewegt werden, ist also nicht als vollständig stationär anzusehen wie in den weiter oben beschriebenen Festbettsystemen. Gleichwohl liegt der Katalysator in Form einer Katalysatorschüttung vor und ist nicht fluidisiert. Diese Zwischenstellung zwischen einer stationären Katalysatorschüttung (Festbett) und einem

fluidisierten, d. h. permanent in wirbelnder Bewegung gehaltenen Katalysatorbett (Wirbelschicht) wird im Folgenden als quasi-stationär (Wanderbett) bezeichnet.

Solche Reaktoren sind in ihrer grundsätzlichen Funktionsweise schon seit langem bekannt. Bereits in den 30er Jahren des vergangenen Jahrhunderts wurden geeignete Systeme für Gasphasenprozesse beschrieben (siehe US-A-1 982 099 und US-A-1 995 293). Heute werden Wanderbettreaktoren insbesondere in der petrochemischen Industrie eingesetzt, wie eine Vielzahl von Patentanmeldungen zeigt. Es handelt sich hierbei um Veredelungsprozesse von Kohlenwasserstoffen, häufig auch unter Einsatz von Wasserstoff.

So beschreibt US-A-3 647 680 ein Verfahren zum kontinuierlichen Betreiben eines Reforming-Regenerations-Prozesses, worin ein Gemisch aus Kohlenwasserstoffen und Wasserstoff ein wanderndes Katalysatorbett lateral durchströmt (sog. "*Radialwanderbettreaktor*") und worin verbrauchter Katalysator regeneriert und in den Prozess zurückgeführt werden kann, ohne diesen unterbrechen zu müssen.

US-A-4 133 743 befasst sich ebenfalls mit der Umsetzung von Kohlenwasserstoffen mit Wasserstoff in einem Radialwanderbettreaktor. Explizit genannt werden Hydroreforming-Prozesse und Reaktionen, die zu aromatischen Kohlenwasserstoffen führen. Es werden mindestens zwei Reaktoren in Serie geschaltet, wobei der einem Reaktor entnommene Katalysator dem nächsten zugeführt wird. Erst nach dem letzten Reaktor findet eine Regeneration des Katalysators statt. Das Verfahren ermöglicht die Einstellung einer relativ konstanten Katalysatoraktivität ohne den Prozess unterbrechen zu müssen.

US-A- 4 188 283 beschreibt eine Anfahrprozedur für die Hydrierung olefinischer Materialien, in der es um die Einstellung der Zu- und Abfuhrmengen bzw. -raten von Katalysator geht.

CN-A-1454970 befasst sich mit dem Problem des Anhaftens/Verklebens von Katalysator und einer gleichmäßigeren axialen Temperaturverteilung. Wasserstoff wird im Unterschuss eingesetzt (molares Verhältnis $H_2$ : Kohlenwasserstoffen =1 : 3).

CN-A-1 333 084 beschreibt den Einbau eines zusätzlichen Leitbleches im unteren Teil des Katalysatorbettes zur Vergleichmäßigung des Katalysatortransportes.

In US-A-2006/0063957 wird der Einsatz eines Radialwanderbettreaktors für die Herstellung von Propylen beschrieben. Die Reaktivitätsunterschiede innerhalb des beweglichen Katalysatorbettes werden dadurch vermindert, dass der entnommene Katalysator nur teilweise regeneriert und mit unregeneriertem vermischt zurückgeführt wird.

Ferner sind auch Wanderbettreaktoren mit einer Vielzahl von Reaktionszonen innerhalb eines Reaktors (sog. "*multiple-stage reactor*") bekannt, siehe z. B. US-A-3 706 536 und EP-A-0 154 492.

Die Durchführung von Reaktionen mit extremer Reaktionsenthalpie wie die Hydrierung aromatischer Nitroverbindungen ist nicht Gegenstand der genannten Anmeldungen zu Wanderbettreaktoren. Sehr stark exo- oder endotherme Reaktionen sind auch in Wanderbettreaktoren nicht leicht zu kontrollieren, und es hat, genau wie in den Festbettsystemen auch, nicht an Ansätzen gefehlt, die Wärmeführung beherrschbar zu machen.

So beschreibt US 2006/0122446 A1 spezielle Axial- oder Radialreaktoren mit beweglichen Katalysatorbetten für Reaktionen mit großer Reaktionsenthalpie. Die dort beschriebenen Reaktoren sind in zwei Reaktionszonen unterteilt, wobei durch Zumischung frischen oder regenerierten Katalysators vor jeder Zone die Reaktivitätsunterschiede zwischen beiden ausgeglichen werden. Die Reaktorkonstruktion ist also relativ kompliziert. Zudem ist in bestimmten Ausführungsformen ein integrierter Wärmeaustauscher zwischen den beiden Reaktionszonen vorgesehen, was darauf schließen lässt, dass bei Reaktionen mit extrem stark exothermem Charakter auf eine Thermostatisierung nicht verzichtet werden kann. Die Thermostatisierung von Reaktoren kann bereits in Festbettsystemen problematisch sein, wenn der Produktionsmaßstab sehr groß ist. In Wanderbettsystemen ist die Thermostatisierung von Reaktoren zwar nicht unmöglich, wie diese Anmeldung zeigt, aber naturgemäß noch komplizierter als in Festbettsystemen.

In US 2006/0115387 A1 ist ein integrierter Wärmeaustauscher in allen Ausführungsformen vorgesehen.

Die Aufgabe der vorliegenden Erfindung war es daher, ein Verfahren zur Herstellung von aromatischen Aminen zur Verfügung zu stellen, worin das rohe Amin mit einer sehr hohen, nur geringen Schwankungen unterworfenen Selektivität hergestellt werden kann. Die Aufgabe konnte gelöst werden durch ein Verfahren zur Hydrierung von Nitroaromaten zu aromatischen Aminen in der Gasphase an in stationären oder quasi-stationären Betten angeordneten Katalysatoren, bei dem durch kontinuierlichen oder periodischen Austausch mindestens eines Teils des eingesetzten Katalysators die Aktivität des zu einem gegebenen Zeitpunkt aktiv am Hydrierprozess beteiligten Katalysators so eingestellt wird, dass die Bildung des aromatischen Amins mit hoher, nur geringen Schwankungen unterworfener Selektivität ermöglicht wird. In bevorzugten Ausführungsformen werden die Aktivität des aktiv am Hydrierprozess beteiligten Katalysators sowie die Selektivität, mit der das gewünschte aromatische Amin gebildet wird, während des gesamten Hydrierprozesses innerhalb enger Grenzen gehalten.

Die Erfindung betrifft ein Verfahren zur Herstellung von aromatischen Aminen der Formel

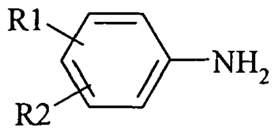

(I)

in der R1 und R2 unabhängig voneinander Wasserstoff, Methyl oder Ethyl bedeuten, wobei R1 zusätzlich Amino bedeuten kann, durch Hydrierung von Nitroaromaten der Formel

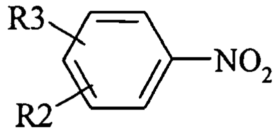

(II)

in der R2 und R3 unabhängig voneinander Wasserstoff, Methyl oder Ethyl bedeuten, wobei R3 zusätzlich Nitro bedeuten kann,
in der Gasphase mit Wasserstoff an in stationären oder quasi-stationären Betten angeordneten Katalysatoren in einem Reaktor, **dadurch gekennzeichnet, dass** der in dem Reaktor befindliche Katalysator kontinuierlich oder in periodischen Abständen zumindest teilweise ausgetauscht wird, wobei innerhalb von 20 Tagen mindestens 10 % des Katalysators ausgetauscht werden.

Die Erfindung betrifft insbesondere ein Verfahren zur Herstellung von aromatischen Aminen der Formel

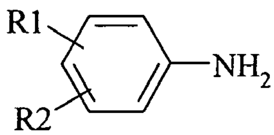

(I)

in der R1 und R2 unabhängig voneinander Wasserstoff, Methyl oder Ethyl bedeuten, wobei R1 zusätzlich Amino bedeuten kann, durch Hydrierung von Nitroaromaten der Formel

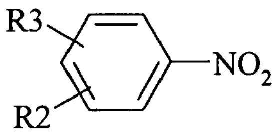

(II)

in der R2 und R3 unabhängig voneinander Wasserstoff, Methyl oder Ethyl bedeuten, wobei R3 zusätzlich Nitro bedeuten kann,
in der Gasphase mit Wasserstoff an in stationären oder quasi-stationären Betten angeordneten Katalysatoren in einem Reaktor, **dadurch gekennzeichnet, dass** der in dem Reaktor befindliche Katalysator kontinuierlich oder in periodischen Abständen zumindest teilweise ausgetauscht wird, wobei
bei kontinuierlichem Katalysatoraustausch innerhalb von Zeitspannen von jeweils 20 Tagen ab Beginn der Hydrierung und
bei Katalysatoraustausch in periodischen Abständen innerhalb von 20 Tagen nach Beginn eines jeden Betriebszeitraums, wobei ein Betriebzeitraum die Zeitspanne zwischen zwei zumindest teilweise durchgeführten Katalysatoraustausch-Vorgängen, inklusive der Erstbefüllung des Reaktors, ist,
mindestens 10 % des Katalysators ausgetauscht werden.

Unter "*kontinuierlichem,* zumindest teilweisen Austausch von Katalysator" wird im Rahmen der vorliegenden Erfindung verstanden, dass permanent Katalysator aus dem Reaktor ausgetragen und durch zugeführten Katalysator ersetzt wird. Bei dieser Fahrweise wird die gesamte Betriebszeit eines Reaktors in Abschnitte zu je 20 Tagen unterteilt, und innerhalb eines jeden dieser Abschnitte werden jeweils mindestens 10 % des Katalysators ausgetauscht. Der kontinuierliche Katalysatoraustausch ist zu unterscheiden vom kontinuierlichen Betrieb des Hydrierprozesses.

Unter "zumindest teilweisem Austausch von Katalysator *in periodischen Abständen*" wird im Rahmen der vorliegenden Erfindung verstanden, dass spätestens 20 Tage nach Beginn der Hydrierung zum ersten Mal mindestens 10 % des Katalysators ausgetauscht werden, dann wiederum spätestens 20 Tage nach diesem Katalysatorwechsel usw.

Die Angabe "mindestens 10 % des Katalysators" kann dabei sowohl "Massen-% bezogen auf die Gesamtmasse des in stationärem oder quasi-stationärem Bett angeordneten Katalysators" als auch "% des Schüttvolumens bezogen auf das gesamte Schüttvolumen des in stationärem oder quasi-stationärem Bett angeordneten Katalysators" bedeuten. Dabei werden 10 % bis 100 %, bevorzugt 20 % bis 90 %, besonders bevorzugt 30 % bis 80 % und ganz besonders bevorzugt 50 % bis 70 % des Katalysators ausgetauscht.

In einer bevorzugten Ausführungsform des Verfahrens wird der aus dem Reaktor entnommene Katalysator ersetzt durch Katalysator, dessen Aktivität so beschaffen ist, dass spätestens 24 Std., bevorzugt spätestens 12 Std., besonders bevorzugt spätestens 6 Std. und ganz besonders bevorzugt spätestens 3 h nach dem Katalysatorwechsel eine momentane Selektivität erzielt wird, welche mindestens 99,0 %, bevorzugt mindestens 99,5 % und besonders bevorzugt mindestens 99,9 % der im letzten Betriebszeitraum vor dem Katalysatorwechsel erzielten durchschnittlichen Selektivität beträgt, wobei unter Betriebszeitraum jeweils die Zeitspanne zwischen zwei Katalysatorwechsel-Vorgängen verstanden wird.

Bevorzugte Nitroaromaten für das erfindungsgemäße Verfahren sind solche der Formel

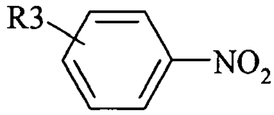

(III)

in denen R3 die oben genannte Bedeutung hat. Besonders bevorzugt als Nitroaromat ist Nitrobenzol (R3 = H).

Die Eindosierung des Nitroaromaten kann so erfolgen, wie in DE-OS-1 809 711 beschrieben, bevorzugt wird jedoch der Nitroaromat im Frischwasserstoff vollständig verdampft und dann gasförmig in den Kreisgasstrom gegeben. Der Vorteil dieser Verfahrensweise liegt in der deutlich geringeren Bildung von Ablagerungen im Reaktor und in den Zuleitungen. Die Verdampfung kann nach dem Stand der Technik in bekannten Verdampfern erfolgen, wie z. B. Fallfilm-, Steigrohr-, Einspritz-, Dünnschicht-, Umlauf- und Wendelrohrverdampfern. Der Verdampfung kann eine grundsätzlich bekannte Tröpfchenabscheidung nachgeschaltet werden. Der Eduktgasstrom wird in bekannter Weise mittels entsprechender Zuführung und Verteilung und/oder durch Vermischungseinrichtungen im Kreisstrom vermischt.

Weiterhin ist die Verdüsung des flüssigen Nitroaromaten in den Frischwasserstoff- oder Kreisgaswasserstoffstrom mittels Einstoff- oder Zweistoffdüsen möglich, wobei die Vereinigung des Eduktgasstromes nach einer Überhitzung in einem Wärmeaustauscher erfolgen kann.

Insbesondere bei der Verdüsungsfahrweise hat es sich als vorteilhaft erwiesen, in Anströmrichtung vor der flächigen Katalysatorschicht eine zusätzliche Schicht aus einem inerten Material anzubringen. Dies hat zum Vorteil, dass bei der Verdüsung evtl. nicht verdampfte Tropfen der eingesetzten Nitroverbindung abgeschieden und weiter verdampft werden können, bevor diese mit der Katalysatorschicht in Kontakt kommen. Auch wird der Katalysator so vor evtl. vorhandenen Verunreinigungen in der aromatischen Nitroverbindung, z. B. hochsiedenden organischen Nebenkomponenten oder Salzen, geschützt. Als inerte Materialien kommen bspw. Packungen aus Stahlwollgewebe oder auch Schüttungen aus Aluminiumoxid mit geringer BET-Oberfläche in Frage. In letzterem Fall ist der Partikeldurchmesser im Vergleich zu den Katalysatorteilchen selbst bevorzugt um den Faktor 1,5 bis 100 größer. Die verwendeten Partikel können wahlweise noch mit einem Oxidationskatalysator, bevorzugt Oxiden des Vanadiums, imprägniert werden. In bevorzugten Ausführungsformen sind die zusätzlichen Schichten aus inerten Materialen so angeordnet, dass sie ausgetauscht werden können, ohne den Hydrierprozess zu unterbrechen.

Das erfindungsgemäße Verfahren kann prinzipiell auf jede beliebige Reaktorgeometrie und Fahrweise angewandt werden. In einer besonders wirtschaftlichen und daher bevorzugten Ausführungsform wird die Aktivität des Katalysators während des gesamten Hydrierprozesses im Mittel auf einem solchen Niveau gehalten, dass der Umsatz an Nitroaromaten, ggf. mit Ausnahme einer Anfahrphase zu Beginn der Hydrierung und kurzzeitiger Störungen des idealen Betriebsablaufs, zu keinem Zeitpunkt unter 99,9000 % sinkt.

Dies wird bevorzugt erreicht durch ein Verfahren, worin der Katalysator im Reaktor in Form eines oder mehrerer quasi-stationärer Katalysatorbetten angeordnet ist,

(i) die Katalysatorbetten in Form von einer oder mehreren regelmäßig geformten flächigen Katalysatorschichten im Reaktor angeordnet sind (bevorzugt wird dabei eine homogene Verweilzeitverteilung des sie durchströmenden Gases gewährleistet),

(ii) die Entnahme von Katalysator aus einer flächigen Katalysatorschicht und die Zuführung von Katalysator in eine flächige Katalysatorschicht ohne Unterbrechung des Hydrierprozesses kontinuierlich oder in periodischen Abständen erfolgen,
(iii) die flächige Katalysatorschicht bzw. die erste von mehreren in Reihe geschalteten flächigen Katalysatorschichten mit einem Gasgemisch angeströmt wird, das pro Mol Nitrogruppe 3 Mol bis 150 Mol Wasserstoff enthält,
(iv) die Hydrierung bei einem absoluten Druck von 1 bar bis 50 bar, bei einer Eingangstemperatur des eingesetzten Gasgemisches von 150 °C bis 400 °C und einer maximalen Katalysatortemperatur von 600 °C unter adiabaten Bedingungen durchgeführt wird,
(v) aus dem bei der Hydrierung erhaltenen Reaktionsgemisch Wasserstoff abgetrennt und der so erhaltene Wasserstoff in die Hydrierung zurückgeführt wird.

Diese bevorzugte Ausführungsform des erfindungsgemäßen Verfahrens zeichnet sich insbesondere dadurch aus, dass Zufuhr und Entnahme des Katalysators kontinuierlich oder in periodischen Abständen erfolgen können, ohne dass der Hydrierprozess unterbrochen werden muss, was in dieser Erfindung als Wanderbett bezeichnet wird.

In dieser bevorzugten Ausführungsform des erfindungsgemäßen Verfahrens, der "Wanderbettvariante", nimmt zu einem gegebenen Zeitpunkt nur ein Teil des in einem Reaktor befindlichen Katalysators aktiv am Hydrierprozess teil, d. h. übt eine katalytische Wirkung aus (in Fig. 1a mit 3b bezeichnet). Nur dieser zu einem gegebenen Zeitpunkt gerade aktiv am Hydrierprozess beteiligte Katalysator ist in regelmäßig geformten flächigen Schichten angeordnet. Aus diesen regelmäßig geformten flächigen Schichten wird laufend oder periodisch, bevorzugt periodisch, ein bestimmter Anteil des aktiv am Hydrierprozess beteiligten Katalysators entnommen und durch zugeführten Katalysator ersetzt. Die Menge des entnommenen Katalysators, Austauschfrequenz und Beschaffenheit des zugeführten Katalysators werden dabei bevorzugt so gewählt, dass der Umsatz an Nitroaromaten, ggf. mit Ausnahme einer Anfahrphase zu Beginn der Hydrierung und kurzzeitiger Störungen des idealen Betriebsablaufs, zu keinem Zeitpunkt unter 99,9000 %, bevorzugt unter 99,9900 %, besonders bevorzugt unter 99,9990 %, sinkt. Daher kann der Hydrierprozess in dieser Ausführungsform des erfindungsgemäßen Verfahrens, abgesehen von unvermeidbaren Unterbrechungen des Produktionsprozesses bspw. durch gesetzlich vorgeschriebene Wartungen und Inspektionen, vollkontinuierlich betrieben werden. Der Ausdruck "zu Beginn der Hy-drierung" bezieht sich in dieser Ausführungsform demnach auf die Erstinbetriebnahme des Reaktors und Wiederinbetriebnahmen nach solchen Unterbrechungen.

In einer besonders bevorzugten Ausführungsform der Wanderbettvariante des erfindungsgemäßen Verfahrens ist die Ausdehnung der flächigen Katalysatorschichten in Strömungsrichtung des Eduktgasgemisches ($L_E$) kleiner als die Ausdehnung in Richtung des Katalysatoraustrags ($L_K$). Hierbei beträgt $L_E$ zwischen 1 cm und kleiner 100 cm, bevorzugt zwischen 2 cm und kleiner 50 cm und besonders bevorzugt zwischen 5 cm und kleiner 25 cm. $L_K$ ist in dieser Ausführungsform immer größer als $L_E$ und beträgt bevorzugt maximal 20 m, besonders bevorzugt maximal 10 m und ganz besonders bevorzugt maximal 5 m. Die Fließrichtung des Katalysators und die Fließrichtung des Eduktgasgemisches liegen bevorzugt senkrecht zueinander, wie in Fig. 1a und 1b gezeigt. Abweichungen vom idealen rechten Winkel im Bereich von $\pm$ 20 % sind auch möglich.

Die flächigen Katalysatorschichten werden bevorzugt zwischen gasdurchlässigen Wandungen angebracht. Bevorzugt handelt es sich hierbei um jeweils zwei metallische Roste oder Siebe in Form von Hohlzylindern, zwischen denen sich der Katalysator befindet. Zusätzlich können vor, hinter oder vor und hinter den Katalysatorschichten technische Vorrichtungen zur gleichmäßigen Gasverteilung angebracht werden. Dies können bspw. Lochplatten, Glockenböden, Ventilböden oder andere Einbauten sein, die durch Erzeugung eines hinreichend großen, gleichmäßigen Druckverlusts einen gleichförmigen Eintritt des Gases in die Katalysatorschüttung bewirken. (Die Bezeichnungen "vor der Katalysatorschicht" und "hinter der Katalysatorschicht" beziehen sich in dieser Anmeldung immer auf die Strömungsrichtung des Eduktgases.)

Die flächigen Katalysatorschichten können in dem erfindungsgemäßen Verfahren in einem Reaktor oder in mehreren Reaktoren angeordnet sein. Ein Reaktor kann im erfindungsgemäßen Verfahren eine Katalysatorschicht oder mehrere Katalysatorschichten enthalten. Mehrere Reaktoren mit einer Katalysatorschicht können also durch eine geringere Anzahl an Reaktoren mit mehreren Katalysatorschichten ersetzt werden.

Mehrere Katalysatorschichten in einem Reaktor können übereinander oder nebeneinander angeordnet sein. In beiden Fällen sind alle Katalysatorschichten bevorzugt mit Vorrichtungen zur Zugabe und Entnahme von Katalysator ausgestattet. Sind mehrere Katalysatorschichten in einem Reaktor übereinander angeordnet, so wird die oberste Katalysatorschicht mit Katalysator von außerhalb des Reaktors beschickt, während die übrigen Katalysatorschichten bevorzugt mit dem Katalysator beschickt werden, welcher der nächst höhergelegenen Katalysatorschicht zuvor entnommen wurde. Sind mehrere Katalysatorschichten in einem Reaktor nebeneinander angeordnet, so wird bevorzugt jede mit Katalysator von außerhalb des Reaktors beschickt.

Beim Einsatz mehrerer Reaktoren können diese in Reihe oder parallel angeordnet sein.

Bevorzugt wird in dem erfindungsgemäßen Verfahren wenigstens ein Reaktor adiabat betrieben. Dabei werden bevorzugt maximal 10, besonders bevorzugt maximal 5, ganz besonders bevorzugt maximal 3 solcher Reaktoren hintereinander angeordnet. Jeder der in Reihe geschalteten Reaktoren kann durch mehrere parallel geschaltete ersetzt werden. Dabei werden bevorzugt maximal 5, besonders bevorzugt maximal 3, ganz besonders maximal 2 Reaktoren

parallel geschaltet. Das erfindungsgemäße Verfahren umfasst demnach bevorzugt maximal 50 und minimal 1 Reaktor.

Die Zahl der Katalysatorschichten in einem Reaktor liegt bevorzugt zwischen 1 und 10, besonders bevorzugt zwischen 1 und 5 und ganz besonders bevorzugt zwischen 1 und 3.

Die Figuren 1a und 1b zeigen schematisch einen für die Durchführung des erfindungsgemäßen Verfahrens geeigneten Reaktor. Schleusenvorrichtungen und Ähnliches wurden der Übersichtlichkeit halber weggelassen. Figur 1a zeigt einen Schnitt in Längsrichtung, Figur 1b zeigt einen Schnitt in Querrichtung für den gleichen Reaktor wie in Figur 1a. Es bedeuten:

(1) Reaktor
(2) Edukteinlass (graue Pfeile symbolisieren den Fluss der Gasmischung),
(3a) Katalysator, der dem Hydrierprozess zugeführt werden kann,
(3b) aktiv am Hydrierprozess teilnehmender, in flächenförmiger Schicht angeordneter Kata- lysator,
(3c) Katalysator, der aus dem Reaktor ausgeschleust werden kann,
(4) gasdurchlässige Wandungen,
(5) Produktauslass,
(6) Katalysatoreinlass (schwarze Pfeile symbolisieren die Fließrichtung des Katalysators),
(7) Katalysatorauslass,
$L_E$ die Länge der flächigen Katalysatorschichten in Richtung (Strömungsrichtung) des Eduktgasstromes,
$L_K$ die Länge der flächigen Katalysatorschichten in Richtung des Katalysatoraustrags.

In der gezeigten Ausführungsform fließt die Gasmischung zunächst von außen nach innen durch die Katalysatorschicht und dann nach oben aus dem Reaktor. Andere Möglichkeiten der Gasführung (etwa von innen nach außen und dann nach oben oder von innen nach außen und dann nach unten oder von außen nach innen und dann nach unten) sind ebenfalls denkbar.

Bevorzugt wird das Gasgemisch vor Beginn der Hydrierung an den Katalysatorschichten homogenisiert, d. h. beispielsweise in einem statischen Mischer vermischt. Die flächigen Katalysatorschichten bzw. die erste von mehreren in Reihe geschalteten flächigen Katalysatorschichten werden mit einem Gasgemisch angeströmt, das bevorzugt pro Mol Nitrogruppe 3 Mol bis 150 Mol, besonders bevorzugt 6 Mol bis 125 Mol, ganz besonders bevorzugt 12 Mol bis 100 Mol, ganz besonders bevorzugt 50 Mol bis 90 Mol Wasserstoff, enthält. In einer bevorzugten Ausführungsform enthält das Eduktgasgemisch darüber hinaus pro Mol Nitrogruppe noch 0,01 Mol bis 100 Mol, besonders bevorzugt 3 Mol bis 50 Mol, ganz besonders bevorzugt 4 Mol bis 25 Mol Wasser. Das Vorhandensein von Wasser im Eduktgasstrom hat sich als vorteilhaft erwiesen, weil es eine Verzögerung der Desaktivierung des Katalysators durch Verkokung zur Folge hat, wodurch eine verringerte Austauschfrequenz möglich wird. Wassermoleküle konkurrieren erfolgreich mit organischen Molekülen um die freien Zentren auf der Katalysatoroberfläche, wodurch die Verweilzeit der organischen Moleküle herabgesetzt und somit der Desaktivierungsprozess verlangsamt wird.

Das in den Reaktor eintretende Gasgemisch hat eine bevorzugte Eingangstemperatur von 150°C bis 400 °C, besonders bevorzugt 200 °C bis 300 °C und ganz besonders bevorzugt 220 °C bis 280 °C. Aufgrund des stark exothermen Charakters der Hydrierung von aromatischen Nitroverbindungen kommt es in der Katalysatorschicht zu einem adiabaten Temperatursprung. Die Prozessparameter werden bevorzugt so gewählt, dass in den Katalysatorschichten maximal Temperaturen bis 600 °C, bevorzugt bis maximal 550 °C und besonders bevorzugt bis maximal 500 °C auftreten.

Die Hydrierung wird bevorzugt bei einem absoluten Druck von 1 bar bis 50 bar, besonders bevorzugt 2 bar bis 20 bar und ganz besonders bevorzugt 2 bar bis 10 bar durchgeführt.

In einer bevorzugten Ausführungsform wird das Reaktionsgemisch nach Verlassen einer Katalysatorschicht zunächst unter Dampfgewinnung (bevorzugt Wasserdampf) abgekühlt. Dazu leitet man es bevorzugt durch einen oder mehrere Wärmeaustauscher. Dies können die dem Fachmann bekannten Wärmeaustauscher, wie z. B. Rohrbündel-, Platten-, Ringnut-, Spiralstrom-, Rippenrohr-Wärmeaustauscher sein. Soll das Reaktionsgemisch noch weitere Katalysatorschichten durchströmen, so erfolgt diese Abkühlung bevorzugt auf die Eingangstemperatur der nächsten Katalysatorschicht ohne Kondensation des gebildeten aromatischen Amins. Bevorzugt nur nach dem Durchströmen der letzten Katalysatorschicht wird das Gasgemisch auch soweit abgekühlt, dass aromatisches Amin aus dem Reaktionsgemisch durch Kondensation entfernt werden kann. In dieser Ausführungsform wird dann nur die erste Katalysatorschicht mit einem Gasgemisch angeströmt, das pro Mol Nitrogruppe 3 Mol bis 150 Mol Wasserstoff sowie ggf. 0,01 Mol bis 100 Mol Wasser enthält. Die nächste Katalysatorschicht wird dann bereits mit dem aus der vorigen Katalysatorschicht erhaltenen, ggf. beispielsweise mit frischem Wasserstoff und frischem Nitroaromaten aufbereiteten Gasgemisch angeströmt. Es ist jedoch auch die Ausschleusung einzelner Komponenten oder die Zufuhr anderer bzw. weiterer Komponenten zwischen den Katalysatorschichten möglich.

Wird im Fall von mehreren in Reihe geschalteten Reaktoren nicht nur nach dem letzten, sondern nach jedem Reaktor das aromatische Amin durch Kondensation abgetrennt, so wird bevorzugt jeder der Reaktoren mit einem Gasgemisch angeströmt, das pro Mol Nitrogruppe 3 bis 150 Mol Wasserstoff sowie ggf. 0,01 Mol bis 100 Mol Wasser enthält.

Die Rezyklierung des Wasserstoffs erfolgt bevorzugt in der Weise, dass nach Abtrennung der kondensierbaren Bestandteile des Reaktionsgemisches Wasserstoff und ggf. auch Inertgas (bevorzugt Stickstoff) sowie ggf. Wasserdampf in den Hydrierprozess zurückgeführt werden.

Der Kreisgasstrom durchläuft bevorzugt einen oder mehrere Kompressor(en), um den Strömungswiderstand von Reaktoren und Wärmeaustauschern auszugleichen und den Massenstrom des Kreisgases zu steuern. Die Kompressoren können einfache, bekannte Maschinen (z. B. Flüssigkeitsringpumpen, Drehkolbengebläse, Turbogebläse oder -verdichter) sein, da der Druckverlust durch die Bauweise der Reaktoren klein gehalten werden kann. Bevorzugt werden trocken laufende Verdichter benutzt.

Bevorzugt wird das Kreisgas unmittelbar vor der ersten Katalysatorschicht mittels eines Wärmeaustauschers wieder auf die Eingangstemperatur von 150 °C bis 400 °C gebracht. Davor oder danach, bevorzugt danach, werden Nitroaromat und Frischwasserstoff wie oben beschrieben eindosiert, sowie ggf. Wasser und Inertgas zugeführt.

In einer besonders wirtschaftlichen Variante des Verfahrens wird das gasförmig anfallende Reaktionswasser bevorzugt nur unvollständig auskondensiert und der verbliebene Wasserdampf zusammen mit dem restlichen Kreisgas zurückgeführt, sodass auf eine externe Zugabe von Wasser verzichtet werden kann.

Das Kondensat wird bevorzugt in eine technische Einrichtung zur Trennung flüssiger Phasen geleitet, und die wässrige und die organische Phase werden getrennt aufgearbeitet. Aus der wässrigen Phase gewonnenes aromatisches Amin wird der Aufarbeitung der organischen Phase zugeführt. Die Aufarbeitungen erfolgen in bekannter Weise durch Destillation bzw. durch Strippen mit Wasserdampf. Durch die Vorzüge des erfindungsgemäßen Verfahrens (gleichmäßige Selektivität auf hohem Niveau) gestaltet sich die Aufarbeitung gegenüber anderen Verfahren als einfach.

Als Katalysatoren können prinzipiell alle bisher für die Gasphasenhydrierung von Nitroverbindungen beschriebenen Kontakte eingesetzt werden. In der bevorzugten Ausführungsform des Wanderbettreaktors ist darüber hinaus von Bedeutung, dass Morphologie und mechanische Belastbarkeit der eingesetzten Katalysatoren die kontinuierliche oder periodische Zufuhr und Entnahme von Katalysator erlauben. Solche Katalysatoren enthalten bspw. die weiter oben genannten Elemente, entweder als Legierung oder als Mischoxide und gegebenenfalls auf inertem Trägermaterial. Als Trägermaterialien kommen besonders in Frage: $\alpha$- und $\gamma$-$Al_2O_3$, $SiO_2$, $TiO_2$, $Fe_2O_3/Al_2O_3$-Mischungen und $CuO/Cr_2O_3$-Mischungen. Es können aber prinzipiell auch andere Träger eingesetzt werden.

Prinzipiell können die Trägermaterialien jede beliebige Form aufweisen. In der bevorzugten Ausführungsform des Wanderbettreaktors ist darüber hinaus von Bedeutung, dass die Rieselfähigkeit des Materials gewährleistet ist. Bevorzugt werden solche Trägermaterialien eingesetzt, deren Morphologie im Wesentlichen kugelförmig ist. Der Kugeldurchmesser der im erfindungsgemäßem Verfahren einsetzbaren Träger liegt zwischen 0,01 mm und 10 mm, bevorzugt zwischen 0,1 mm und 8 mm, besonders bevorzugt zwischen 0,5 mm und 4 mm und ganz besonders bevorzugt zwischen 1 mm und 2 mm.

In der bevorzugten Ausführungsform des Wanderbettreaktors ist die Abriebbeständigkeit des Trägermaterials von großer Wichtigkeit. Daher werden in dem erfindungsgemäßen Verfahren bevorzugt solche Trägermaterialien eingesetzt, deren Bruchkraft im Mittel größer als 30 N, bevorzugt größer als 60 N, besonders bevorzugt größer als 80 N und ganz besonders bevorzugt größer als 90 N ist, wobei der Wert für die Bruchkraft ein Mittelwert aus mindestens 100 Messungen an einzelnen Kugeln ist (Messung in Anlehnung an DIN EN ISO 604, Ausgabe Dezember 2003).

Im erfindungsgemäßen Verfahren ist ebenfalls die BET-Oberfläche des Trägermaterials von großer Wichtigkeit, weil Katalysatoren auf Trägern mit sehr großer BET-Oberfläche zu verstärkter Nebenproduktbildung neigen. Daher werden bevorzugt solche Trägermaterialien eingesetzt, deren BET-Oberfläche weniger als 50 $m^2/g$, bevorzugt weniger als 25 $m^2/g$, besonders bevorzugt weniger als 13 $m^2/g$ und ganz besonders bevorzugt weniger als 7 $m^2/g$ beträgt.

Besonders bevorzugte Trägermaterialien sind Kugeln aus $\alpha$-Aluminiumoxid mit einer BET-Oberfläche von weniger als 7 $m^2/g$ und einer Bruchkraft von größer als 90 N.

Auf dem Trägermaterial sind bevorzugt folgende Aktivstoffklassen niedergeschlagen:

(a) 1 - 100 $g/l_{Katalysator}$ eines oder mehrerer Metalle der Gruppen 8 bis 12 des Periodensystems der Elemente (die Bezeichnung der Gruppen des Periodensystems erfolgt hier und im Folgenden nach der IUPAC-Empfehlung von 1986), und

(b) 0,01 - 100 $g/l_{Katalysator}$ eines oder mehrerer Übergangsmetalle der Gruppen 4 bis 7 und 12 sowie ggf.

(c) 0,01 - 100 $g/l_{Katalysator}$ eines oder mehrerer Hauptgruppenelemente der Gruppen 13 bis 15.

Elemente der Gruppe 12 können somit ggf. als Aktivstoffe (a) und (b) wirken. Bevorzugte Aktivstoffe sind Pd als Metall (a), Ti, V, Nb, Ta, Cr, Mo, W, Re als Übergangsmetall (b) und Ga, Pb, Bi als Hauptgruppenelemente (c).

Die Aktivstoffe werden bevorzugt in Form ihrer löslichen Salze auf den Träger gebracht, gegebenenfalls sind mehrere Behandlungen (Tränkungen) pro Komponente erforderlich.

Es hat sich weiterhin als vorteilhaft erwiesen, die genannten Katalysatoren zusätzlich mit einer schwefelhaltigen oder phosphorhaltigen, bevorzugt phosphorhaltigen Verbindung zu dotieren. Ein solcher zusätzlicher Gehalt an Dotierungsmittel beträgt bevorzugt 0,001 - 2 Massen-%, bevorzugt 0,01 - 1 Massen-% Schwefel oder Phosphor, bevorzugt

Phosphor, in chemisch gebundener Form, bezogen auf die Gesamtmasse des Katalysators. Als phosphorhaltige Verbindungen für die Dotierung der erfindungsgemäßen Katalysatoren sind bevorzugt zu nennen: die Sauerstoffsäuren des Phosphors $H_3PO_4$, $H_3PO_3$, $H_3PO_2$ oder deren Alkalisalze, wie z. B. Natriumdihydrogenphosphat, Natrium- oder Kaliumphosphat oder Natriumhypophosphit. Als schwefelhaltige Verbindungen kommen bevorzugt Salze der Sauerstoffsäuren des Schwefels in Frage, besonders bevorzugt sind die Alkalisalze der Schwefelsäure.

Geeignete Katalysatoren sind bspw. die in DE-OS 2 849 002 oder EP 1 882 681 A1 beschriebenen. Die in DE-OS 2 849 002 beschriebene Vorbehandlung mit einer Base ist jedoch nicht unbedingt erforderlich.

Die Belastung der Katalysatoren in dem erfindungsgemäßen Verfahren kann sehr hoch sein und kann bevorzugt 0,1 $g_{Nitraromat}/[ml_{Katalysator}\ h]$ bis zu 20 $g_{Nitroaromat}/[ml_{Katalysator} \cdot h]$, besonders bevorzugt bis zu 15 $g_{Nitroaromat}/[ml_{Katalystor} \cdot h]$, ganz besonders bevorzugt bis zu 10 $g_{Nitroaromat}/[ml_{Katalystor} \cdot h]$ und insbesondere ganz besonders bevorzugt bis zu 5 $g_{Nitroaromat}/[ml_{Katalystor} \cdot h]$ betragen. Im Falle mehrerer Katalysatorschichten kann die Belastung von Schüttung zu Schüttung variiert werden. Bevorzugt liegt die Belastung jedoch in allen Katalysatorschichten in dem Bereich von 0,1 $g_{Nitroaromat}/[ml_{Katalystor} \cdot h]$ bis zu 20 $g_{Nitroaromat}/[ml_{Katalystor} \cdot h]$. Das erfindungsgemäße Verfahren zeichnet sich demnach durch hohe Raum-Zeit-Ausbeuten aus.

Das erfindungsgemäße Verfahren gestattet in der bevorzugten Ausführungsform als Wanderbettverfahren die Entnahme und Zufuhr von Katalysator, ohne den laufenden Hydrierprozess unterbrechen zu müssen.

Die Entnahme von Katalysator erfolgt hierbei bevorzugt in der Weise, dass

- eine gleichmäßige Durchströmung der Katalysatorschicht mit Eduktgas gewährleistet ist, und / oder
- die Verweilzeitverteilung des Eduktgases in der Katalysatorschicht sehr eng ist, und / oder
- ein homogener Transport des Katalysators mit enger Verweilzeitverteilung erreicht wird, und / oder
- Totraumgebiete, d. h. ruhende oder rezyklierende Bereiche der Katalysatorschüttung im Bereich der Eduktgasdurchströmung vermieden werden, und / oder
- ein möglichst mechanisch schonender Katalysatortransport erreicht wird, um Abrieb zu vermeiden.

Dazu wird der Katalysator bevorzugt in einen Sammelbehälter oder Sammelbereich geleitet und von dort zentral über einen geeigneten dosierenden Austrag ausgetragen. Als Austragsorgane kommen Zellenradschleusen, Doppelklappensysteme mit getaktet betriebenen, geeigneten Absperrorganen wie Kugelventilen, Klappen oder Schiebern, Drehzahl-geregelte oder -angepasste Schneckenförderer, Vibrationsrinnen o. Ä. in Frage.

Der Transfer vom Reaktionsraum in den Sammelbereich, der unmittelbar an den Reaktor angeflanscht sein kann, muss möglichst symmetrisch aufgebaut sein, um einen gleichmäßigen Feststofftransport durch alle Reaktorbereiche zu gewährleisten.

Der Übergang vom Reaktions- in den Sammelbehälter kann auch mit Rohren erfolgen, die möglichst äquidistant in einer oder mehreren Rohrreihen entlang des Umfanges der flächigen Katalysatorschicht angeordnet sind und mit gleichem Neigungswinkel und gleicher Rohrlänge symmetrisch in den Einlassbereich des Sammelbehälters geführt werden. Die Rohre sind am Reaktor so weit unterhalb der durchströmten Katalysatorschicht anzuordnen, dass eine Eduktgasdurchströmung der Totraumgebiete des Katalysatortransportes zwischen den Rohreinmündungen am Reaktorboden vermieden wird. Ggf. sind diese Toträume für den Katalysatortransport durch konische Bereiche in den Einlaufbereichen der einzelnen Rohre und/oder durch entsprechende Einbauten auf dem Reaktorboden zu minimieren.

Ggf. erfolgt auch der Zu- oder Ablauf des Edukt- bzw. Produktgases in bzw. aus dem Innenraum des Reaktors auf der Reaktorunterseite. In diesem Fall ist die Variante der Katalysatorableitung über Rohre von Vorteil, da dann das Gas in ein oder mehreren Gasrohren, die zwischen den Katalsatorsammelrohren angeordnet werden können, geführt werden kann.

Die Zufuhr von Katalysator bevorzugt erfolgt in der Weise, dass

- eine gleichmäßige Durchströmung der Katalysatorschicht mit Eduktgas gewährleistet ist, und / oder
- die Verweilzeitverteilung des Eduktgases in der Katalysatorschicht sehr eng ist, und / oder
- ein homogener Transport des Katalysators mit enger Verweilzeitverteilung erreicht wird, und / oder
- Totraumgebiete, d. h. ruhende oder rezyklierende Bereiche der Katalysatorschüttung im Bereich der Eduktgasdurchströmung vermieden werden, und / oder
- ein möglichst mechanisch schonender Katalysatortransport erreicht wird, um Abrieb zu vermeiden.

Dazu wird der Katalysator bevorzugt über einen Verteilerbehälter auf dem Umfang der flächigen Katalysatorschicht verteilt. Der Zulauf sollte symmetrisch erfolgen, z. B. über kegelförmige Einbauten mit einem Neigungswinkel, der den Schüttguteigenschaften, insbesondere dem Rutsch- und Schüttwinkel der Katalysatorpartikel, angepasst ist.

Der Transfer vom Verteilerbehälter in die flächige Katalysatorschicht kann auch über Rohre erfolgen, die konzentrisch am Verteilerbehälter angeordnet sind und in einer oder mehreren Rohrreihen äquidistant entlang des Umfanges der Katalysatorschicht münden. Neigungswinkel und Rohrlängen der einzelnen Rohre sollten übereinstimmen.

Der Einlaufbereich des Katalysators im Reaktionsraum ist bevorzugt so lang auszuführen, dass im Durchströmbereich des Eduktgases eine ausreichende Katalysatorschichthöhe besteht und ein Bypass des Eduktgases über den Gasraum oberhalb der Katalysatorschüttung erschwert ist. Dies wird erreicht durch im Vergleich zur durchströmten Katalysatorschichtdicke lange Strömungswege durch die Katalysatorschüttung.

Die Zuführung über einzelne Rohre eignet sich besonders, wenn die Zu- oder Ableitung des Edukt- bzw. Produktgases in bzw. aus dem Reaktorinnern auf der Reaktoroberseite angeordnet ist. In diesem Fall erfolgt die Gasführung über ein oder mehrere Gasrohre, die zwischen den Katalystorzulaufrohren angordnet werden können.

Die Führung des Katalysators innerhalb des gasdurchströmten Bereichs erfolgt bevorzugt dergestalt, dass

- eine gleichmäßige Durchströmung des Gases mit enger Verweilzeitverteilung durch die dünne, flächige Katalysatorschicht erreicht wird, und / oder
- der Katalysator gleichmäßig und unter möglichst geringer mechanischer Belastung transportiert wird.

Dazu wird der innen- und außenseitige Mantel der flächigen Katalysatorschicht gasdurchlässig ausgeführt, z. B. aus Lochblech, porösem Material, Membranen oder bevorzugt aus Spaltsieben mit in Katalysatorfließrichtung ausgerichteten Spalten und ansonsten glatter Ausführung auf der Katalysatorseite.

Als Fördergas für den Katalysator kommt beispielsweise in Frage: Wasserstoff oder Gemische aus Wasserstoff und Intertgasen oder Intertgase. Bevorzugtes Inertgas ist Stickstoff.

Die Gasdurchströmung erfolgt bevorzugt mit Geschwindigkeiten von 0,1 m/s bis 20 m/s, besonders bevorzugt 0,5 m/s bis 10 m/s und ganz besonders bevorzugt mit 1 m/s bis 3 m/s. Die Gasgeschwindigkeit ist aufgrund der Querschnittsänderung der gekrümmten, kreisförmigen Katalysatorschicht sowie aufgrund der Änderung von Temperatur und Zusammensetzung des Gases in der Reaktionszone im Allgemeinen nicht konstant.

Der entnommene Katalysator (Katalysatoraustrag) durchläuft bevorzugt eine Strippstufe, in der das Reaktionsgas aus den Zwickeln und den Katalysatorträgerpartikeln entfernt wird. Die Strippung erfolgt durch Durchströmung mit einem Inertgas, vorzugsweise Stickstoff.

Der ggf. infolge Katalysatorabriebs anfallende Staub kann mit geeigneten Apparaten wie Sieben, Sichtern, Zyklonen oder Filtern laufend oder periodisch abgetrennt und aus dem Prozess ausgeschleust und entsorgt werden.

Der Katalysatoraustrag wird vorzugsweise zu einem Massenanteil von 1 % bis 100 %, bevorzugt von 70 % bis 100 %, besonders bevorzugt von 90 % bis 100 %, bezogen auf die Gesamtmasse des Katalysatoraustrags, rezykliert.

Der rezyklierte Anteil kann vor der Rückführung in den Reaktor ganz oder teilweise durch Behandlung mit sauerstoffhaltigen Gasgemischen, bevorzugt Luft bzw. Luft-Stickstoff-Gemischen, bei erhöhter Temperatur regeneriert werden, und zwar bevorzugt bei Temperaturen von 100 °C bis 400 °C, besonders bevorzugt bei Temperaturen von 200 °C bis 300 °C. Hierbei kann die Regeneration vollständig (kein signifikanter Restkohlenstoffgehalt mehr) oder auch unvollständig (z. B. für kürzere Zeit und/oder bei geringerer Temperatur als bei der vollständigen Regeneration) durchgeführt werden. Der Massenanteil an regeneriertem Katalysator kann 0,1 % bis 100 %, bevorzugt 1 % bis 50 %, besonders bevorzugt 5 % bis 30 % der insgesamt rezyklierten Katalysatormenge betragen. Der regenerierte Katalysator wird ggf. mit dem unregeneriert zurückgeführtem Katalysatoranteil und/oder zusätzlich -zur Auffüllung und zur Kompensation der aus dem Prozess ausgeschleusten Katalysatormenge- zugeführtem Frischkatalysator und/oder extern aufgearbeitetem Katalysator gemischt. Diese Mischung kann durch statische oder bewegte kontinuierliche Feststoffmischer oder in diskontinuierlich arbeitenden Feststoffmischern erfolgen.

Die so erhaltene Katalysatormischung wird bevorzugt vor der Rückführung in den Prozess erneut gestrippt, um den Sauerstoff aus den Zwickeln und den Katalysatorträgerpartikeln zu entfernen. Dazu wird der Katalysator mit Inertgas durchströmt, vorzugsweise mit Stickstoff.

Die so erhaltene Katalysatormischung durchläuft anschließend eine Aktivierung mit einem Aktivierungsgas, vorzugsweise Wasserstoff. Die Aktivierung erfolgt bei Temperaturen von 100 °C bis 400 °C, bevorzugt von 200 °C bis 300 °C.

Die so erhaltene Katalysatormischung wird ggf. mit dem unregeneriert rezykliertem Katalysator zusammengeführt und gemischt. Diese Zusammenführung kann auch vor der Aktivierung erfolgen, wie oben beschrieben. Anschließend wird die Katalysatormischung in den Prozess zurückgeführt.

Der dem Prozess zuzuführende Katalysator wird mit einer geeigneten Transporteinrichtung zum Sammelbehälter im Katalysatorzulauf des Reaktors transportiert. Dieser Feststofftransport geschieht vorzugsweise per Flugstaubförderung in inerter Atmosphäre. Als Inertgas wird bevorzugt Stickstoff verwendet.

Die Ausschleusung des zu regenerierenden Katalysatoranteils kann je nach Aufstellungsort der Reaktoren für die Regeneration vor oder nach dem Feststofftransport des nicht zu regenerierenden Katalysatoranteils erfolgen.

Der Feststofftransport des regenerierten oder zu regenerierenden Katalysatoranteils kann auch nach der Abtrennung und vor der Zusammenführung getrennt von dem rezyklierten nicht zu regenerierenden Katalysatoranteil erfolgen.

Die Figur 2 zeigt schematisch ein Block-Fließbild einer möglichen Ausführungsform des erfindungsgemäßen

Verfahrens unter Einsatz eines bewegten Katalysatorbetts (Wanderbett). Die Darstellung ist stark vereinfacht.

Es bedeuten:

| | |
|---|---|
| (1) | Reaktor, |
| (3d) | Katalysator, der dem System von außen zugeführt wird, |
| (3e) | aus dem System ausgetragener Katalysatorstaub |
| (3f) | Katalysator, der der Regeneration zugeführt wird, |
| (3g) | Katalysator, der unregeneriert in die Reaktion zurückgeführt wird, |
| (8) | Mischkammer, |
| (9) | Stripper, |
| (10) | Aktivierung, |
| (11) | Stripper, |
| (12) | Staubabscheidung, |
| (13) | Regeneration. |

In einer besonders bevorzugten Ausführungsform der Wanderbettvariante des erfindungsgemäßen Verfahrens wird nur derjenige Anteil des dem Reaktor entnommenen Katalysators, der regeneriert werden soll, einer Strippstufe mit Inertgas, bevorzugt Stickstoff, unterzogen. Die Regeneration dieses Katalysatoranteils wird, auch aus Sicherheitsgründen, bevorzugt diskontinuierlich in Batchfahrweise betrieben. Nach der Regeneration wird der Katalysator erneut mit Inertgas gestrippt und bevorzugt mit Wasserstoff aktiviert. Diese Aktivierung kann aufgrund der diskontinuierlichen Batchfahrweise der Regeneration prinzipiell im gleichen Apparat wie die Regeneration erfolgen. Bevorzugt ist jedoch der Regeneration ein zusätzlicher Apparat zur Aktivierung nachgeschaltet, welcher bevorzugt auch als Lagerbehälter für den regenerierten und aktivierten Katalysator dient. Dieser so vorbereitete Katalysator kann der Reaktion nun wieder zugeführt werden. Zu diesem Zweck wird er ganz oder teilweise mit den unregeneriert zurückgeführten Anteilen sowie ggf. dem System von außen zugeführtem Katalysator, der zuvor bevorzugt ebenfalls durch Behandlung mit Wasserstoff aktiviert wurde, vermischt.

Alternativ kann regenerierter Katalysator auch vor der Aktivierung mit von außen zugeführtem Katalysator vermischt werden, sodass eine gleichzeitige Aktivierung beider möglich ist. Dies kann bspw. so realisiert werden, dass nach der Regeneration von bereits im System befindlichen Katalysator dieser zunächst mit einem Intertgas, bevorzugt Stickstoff, gestrippt und dann ganz oder teilweise in einen nachgeschalteten Apparat zur Aktivierung abgelassen wird. Dem System von außen zuzuführender Katalysator wird im Anschluss daran in den nun unter Inertgas stehenden Regenerationsapparat eingefüllt und kann ganz oder teilweise in den Aktivierungsapparat abgelassen werden. Im Aktivierungsapparat können dann beide zusammen mit Wasserstoff behandelt werden, bevor sie, ggf. nach Zumischung von Anteilen unregeneriert zurückgeführten Katalysators, der Reaktion zugeführt werden. Alternativ kann von außen zuführender Katalysator, bevorzug nach Durchlaufen einer Strippstufe, auch direkt in den Aktivierungsapparat eingeführt werden.

Bevorzugt werden die verschiedenen Katalysatoranteile (regenerierter Katalysator und ggf. von außen zugeführter Katalysator und ggf. unregenerierter Katalysator) vor Einbringung in den Hydrierreaktor gründlich vermischt, woran sich ein Ausblasen von Katalysatorstaub, bevorzugt mit Wasserstoff, anschließen kann.

Die Figur 3 zeigt schematisch ein mögliches Block-Fließbild dieser Ausführungsform des erfindungsgemäßen Verfahrens mit - beispielhaft - drei Reaktoren. Die Darstellung ist der Übersichtlichkeit halber vereinfacht, bspw. sind Schleusen, Ventile, Kreisgasströme, Kondensation und Aufarbeitung des Produkts nicht dargestellt.

Es bedeuten:

| | |
|---|---|
| (1a, 1b, 1c) | Reaktoren, |
| (3d) | Katalysator, der dem System von außen zugeführt wird, |
| (3f) | Katalysator, der der Regeneration zugeführt wird, |
| (3g) | Katalysator, der unregeneriert in die Reaktion zurückgeführt wird, |
| (3h) | Strom aus Katalysatorstaub und Wasserstoff |
| (3i) | Katalysator, der aus dem System ausgetragen wird |
| (8) | Mischkammer |
| (10) | Aktivierung |
| (14) | Entstaubung des Katalysators |
| (15) | Wasserstoffstrom zum Ausblasen von Katalysatorstaub |
| (16) | Taktbehälter |
| (17a, 17b, 17c) | Produktgasgemisch aus den Reaktoren 1a, 1b, 1c |
| (18a, 18b, 18c) | Eduktgasgemisch aus Nitrobenzol, Wasserstoff und ggf. Wasser für die Reaktoren 1a, 1b, 1c |

(fortgesetzt)

| | |
|---|---|
| (19) | Wärmeaustauscher |
| (20) | Fördervorrichtung für Katalysator |
| (21) | Regeneration und Strippung |

Das erfindungsgemäße Verfahren führt dazu, dass das gewünschte Amin mit einer nur geringen Schwankungen unterworfenen permanent hohen Selektivität gebildet wird (siehe Beispiele), was den Aufwand bei der Aufarbeitung erheblich reduziert.

Die Einstellung einer konstant hohen, nur geringen Schwankungen unterworfenen Selektivität wird im erfindungsgemäßen Verfahren durch den kontinuierlich oder periodisch erfolgenden Austausch des Katalysators gewährleistet. Darüber hinaus sind andere früher bereits beschriebene Maßnahmen zur Selektivitätssteigerung, wie der partielle Ersatz des überstöchiometrisch eingesetzten Wasserstoffs durch ein Inertgas wie z. B. Stickstoff, auch auf das erfindungsgemäße Verfahren anwendbar, in der Regel jedoch nicht nötig.

Der besondere Vorteil der bevorzugten Wanderbettvariante des erfindungsgemäßen Verfahrens gegenüber den herkömmlichen Festbettverfahren ohne kontinuierlichen oder periodischen Katalysatoraustausch liegt darin, dass die niedrigen Selektivitäten, die anfangs mit frischem oder vollständig regeneriertem Katalysator nach dem Stand der Technik auftreten und die wie oben beschrieben die Produktaufarbeitung deutlich erschweren, auf einfache Weise vermieden werden können, da im kontinuierlichen Verfahren bevorzugt nur geringe Bereiche des Reaktors mit Katalysator hoher Aktivität betrieben werden. Darüber hinaus besteht die Möglichkeit, die Aktivität des Katalysators und damit auch die Selektivität des Prozesses durch das Rücklaufverhältnis des Katalysators und dem Anteil des regenerierten Katalysators optimal einzustellen. Dadurch wird im gesamten Reaktor mit einem Katalysator mittlerer Aktivität und optimaler Selektivität gearbeitet. Auf diese Weise wird auch die durchschnittliche Selektivität gegenüber dem bisherigen Verfahren verbessert, und die Selektivitätsschwankungen werden auf ein Minimum reduziert. Da sich in der Wanderbettvariante des erfindungsgemäßen Verfahrens die Aufgabe der Standzeitoptimierung durch Verbesserung des Katalysatorzusammensetzung so nicht mehr stellt, besteht überdies die Möglichkeit, den Katalysator noch besser in Richtung hoher Selektivität zu optimieren, wodurch das erfindungsgemäße Verfahren noch weiter verbessert werden kann. Ferner muss die Produktion für die Katalysatorregeneration nicht unterbrochen werden.

## Beispiele

Alle Beispiele wurden mit dem Katalysatorsystem 9 g/l Pd, 9 g/l V, 3 g/l Pb auf α-Aluminiumoxid durchgeführt (DE-OS-28 49 002). Es wurden unterschiedlich gealterte Katalysatoren dieses Katalysatorsystems eingesetzt:

A) bereits in mehreren Produktionszyklen eingesetzter, bis zum Nitrobenzoldurchbruch betriebener und danach kurz regenerierter (d. h. ca. 10 Std. mit Luft bei 290 °C behandelter) Katalysator,
B) bereits in mehreren Produktionszyklen eingesetzter, bis zum Nitrobenzoldurchbruch betriebener und danach nicht regenerierter Katalysator,
C) bereits in mehreren Produktionszyklen eingesetzter, bis zum Nitrobenzoldurchbruch betriebener und danach lange regenerierter (d. h. ca. 24 Std. mit Luft bei 290 °C behandelter) Katalysator sowie
D) frischer Katalysator.

Die Produkte wurden jeweils durch Gaschromatographie untersucht. Prozentangaben im Zusammenhang mit Mischungen verschiedener Katalysatoren sind als % des Schüttvolumens zu verstehen.

Die nachstehend in den Beispielen 1 bis 2 beschriebenen Versuche wurden in einem thermostatisierten Rohrreaktor durchgeführt, der jeweils 100 ml Schüttvolumen Katalysator enthielt. Die Temperatur des Wärmeträgers wurde mit einer bei allen Versuchen gleichen Rate schrittweise von 250 °C auf 300 °C erhöht. Nitrobenzol wurde mit einem molaren Verhältnis von Wasserstoff zu Nitrobenzol von ca. 12,5 : 1 zu Anilin umgesetzt.

### Beispiel 1 (erfindungsgemäß, Festbettvariante - Unterbrechung der Hydrierung für Katalysatorwechsel)

Der Reaktor wurde befüllt mit einer homogenen Mischung aus 20 % Katalysator A und 80 % Katalysator B. Nitrobenzol wurde solange eingespeist, wie der Umsatz nicht unter 99,9900 % sank (130 h; insgesamt 10,95 kg Nitrobenzol; Betriebszeitraum I).

Erster Katalysatorwechsel und nächster Betriebszeitraum:

Der Katalysator wurde vollständig entnommen und durch eine homogene Mischung aus 40 % Katalysator A und 60 % Katalysator B ersetzt. Nitrobenzol wurde solange eingespeist, wie der Umsatz nicht unter 99,9900 % sank (134 h; insgesamt 11,55 kg Nitrobenzol; Betriebszeitraum II).

Zweiter Katalysatorwechsel und nächster Betriebszeitraum:

Der Katalysator wurde vollständig entnommen und zu 100 % durch Katalysator A ersetzt. Nitrobenzol wurde solange eingespeist, wie der Umsatz nicht unter 99,9900 % sank (184 h; insgesamt 15,46 kg Nitrobenzol; Betriebszeitraum III).

Dritter Katalysatorwechsel und nächster Betriebszeitraum:

Der Katalysator wurde vollständig entnommen und durch eine homogene Mischung aus 60 % Katalysator A und 40 % Katalysator B ersetzt. Nitrobenzol wurde solange eingespeist, wie der Umsatz nicht unter 99,9900 % sank (150 h; insgesamt 12,70 kg Nitrobenzol; Betriebszeitraum IV).

Vierter Katalysatorwechsel und nächster Betriebszeitraum:

Der Katalysator wurde vollständig entnommen und durch eine homogene Mischung aus 80 % Katalysator A und 20 % Katalysator B ersetzt. Nitrobenzol wurde solange eingespeist, wie der Umsatz nicht unter 99,9900 % sank (155 h; insgesamt 12,47 kg Nitrobenzol; Betriebszeitraum V).

**Beispiel 2 (Vergleichsbeispiel - kein Katalysatorwechsel, Unterbrechung der Hydrierung für Regeneration im Reaktor)**

Der Reaktor wurde vollständig mit Katalysator C befüllt. Nitrobenzol wurde solange eingespeist, wie der Umsatz nicht unter 99,9900 % sank (342 h; insgesamt 31,57 kg Nitrobenzol; Betriebszeitraum I).

Regeneration und nächster Betriebszeitraum:

Der gebrauchte Katalysator wurde nicht entnommen, sondern im Reaktor 24 Std. lang mit Luft bei 290 °C regeneriert. Nitrobenzol wurde danach wieder solange eingespeist, wie der Umsatz nicht unter 99,9900 % sank (341 h; insgesamt 30,35 kg Nitrobenzol; Betriebszeitraum II).

Die in Beispiel 2 geschilderte Vorgehensweise entspricht dem gängigen Stand der Technik in Festbettreaktoren. Die folgende Tabelle zeigt einen Vergleich der Beispiele 1 und 2.

Tabelle 1: Gegenüberstellung der Resultate aus den Beispielen 1 und 2

| Bsp. | Betriebszeitraum | Dauer/h | $S_M$ / % [a] | | | | | $\Delta S_M$[b] | $S_D$ / % [c] |
|---|---|---|---|---|---|---|---|---|---|
| | | | t = 24 h | t = 48 h | t = 72 h | t = 96 h | t = 120 h | | |
| 1 | I | 130 | 99,93 | 99,94 | 99,94 | 99,94 | 99,94 | 0,01 | 99,94 |
| | II | 134 | 99,93 | 99,94 | 99,93 | 99,94 | 99,94 | 0,01 | 99,94 |
| | III | 184 | 99,60 | 99,84 | 99,90 | 99,92 | 99,92 | 0,32 | 99,85 |
| | IV | 150 | 99,89 | 99,93 | 99,94 | 99,94 | 99,94 | 0,05 | 99,92 |
| | V | 155 | 99,87 | 99,92 | 99,93 | 99,93 | 99,94 | 0,07 | 99,91 |
| | | | | | | | | Gesamtversuch | 99,91 |

(fortgesetzt)

| Bsp. | Betriebszeitraum | Dauer/h | $S_M$ / % [a] | | | | | $\Delta S_M$[b] | $S_D$ / % [c] |
|---|---|---|---|---|---|---|---|---|---|
| | | | t = 24 h | t = 48 h | t = 72 h | t = 96 h | t = 120 h | | |
| 2 | I | 42 | 95,30 | 98,50 | 99,24 | 99,46 | 99,60 | **4,30** | **99,42** |
| | II | 340 | 96,05 | 98,82 | 99,37 | 99,61 | 99,70 | **3,65** | **99,55** |
| | Gesamtversuch | | | | | | | | **99,49** |

Erläuterungen:
[a] $S_M$: Momentane Selektivität zum Zeitpunkt t.
[b] [c] $S_D$: Durchschnittliche Selektivität im angegebenen Betriebszeitraum bzw. im gesamten Versuch.

$$\Delta S_M = [S_M(120\ h) / \%] - [S_M(24\ h) / \%].$$

Bei vergleichbaren Gesamtmengen an eingesetztem Nitrobenzol und gleichen Anforderungen an den Mindestumsatz wurden im Vergleichsbeispiel signifikant niedrigere durchschnittliche Selektivitäten erzielt als im erfindungsgemäßen Beispiel. Die Selektivitätsschwankungen sind im erfindungsgemäßen Beispiel um eine Größenordnung geringer als im Vergleichsbeispiel.

**Beispiel 3 (erfindungsgemäß, Wanderbettvariante)**

Dieser Versuch wurde in einem adiabat betriebenen Reaktor in Kreisgasfahrweise durchgeführt. Der Reaktor bietet die Möglichkeit, Katalysator über ein Schleusensystem ohne Unterbrechung des Hydrierprozesses zuzuführen und zu entnehmen. Außerdem erlaubt ein eingebautes Rührsystem die Durchmischung des Katalysators auch während der Reaktion. Bei einer Eintrittstemperatur des Eduktgasgemisches von ca. 240 °C, einem absoluten Druck von 4 bar, einer Belastung von 1,0 $g_{Nitrobenzol}$ / ($ml_{Kat.}$ · h) und einem molaren Verhältnis von Wasserstoff zu Nitrobenzol von ca. 80 : 1 wurde Nitrobenzol zu Anilin umgesetzt. Der Reaktor wurde mit ca. 530 ml Schüttvolumen einer homogenen Mischung aus 20 % Katalysator B (andere Charge als in Beispiel 1) und 80 % Katalysator C (andere Charge als in Beispiel 2), entsprechend einer Betthöhe von 20 cm, befüllt. Zu bestimmten Zeiten wurden dem Reaktor jeweils ca. 30 ml Katalysator entnommen und durch anderen ersetzt. Teilweise wurde nach der Zugabe von Katalysator jeweils 2 Minuten gerührt, um die Katalysatorschüttung zu homogenisieren. Details enthält Tabelle 2.

Tabelle 2: Bedingungen in Beispiel 3

| Kat.-Wechsel nach | 18 Std. | 21 Std. | 24 Std. | 42 Std. | 45 Std. | 48 Std. | 66 Std. | 72 Std. | 90 Std. | 93 Std. | 113 Std. | 137 Std. |
|---|---|---|---|---|---|---|---|---|---|---|---|---|
| Zugegebener Katalysator (Mischungverhältnis) | B/C (20/80) | B/C (20/80) | B/C (20/80) | B/C (20/80) | B/C (20/80) | B/C (20/80) | B/C (20/80) | B/C (20/80) | B/C (20/80) | B/C (20/80) | B/C (20/80) | B/C (20/80) |
| Gerührt? | Nein | Nein | Nein | Ja | Ja | Ja | Ja | Ja | Ja | Ja | Ja | Ja |
| Kat.-Wechsel nach | 161 Std. | 165 Std. | 185 Std. | 209 Std. | 255 Std. | 300 Std. | 347 Std. | 371 Std. | 395 Std. | 419 Std. | 443 Std. | 467 Std. |
| Zugegebener Katalysator (Mischungsverhältnis) | B/C (20/80) | B/C (20/80) | B/C (10/90) | B/C (0/90) | B/C (10/90) | B/C (10/90) | B/C (10/90) | B/C (10/90) | B/C (10/90) | B/C (10/90) | B/C (10/90) | B/C (10/90) |
| Gerührt? | Ja | Ja | Ja | Ja | Ja | Ja | Ja | Ja | Ja | Ja | Ja | Ja |

Der Versuch wurde nach 491 Std. abgebrochen; eine Fortführung wäre aber ohne Weiteres möglich gewesen. Der mittlere Nitrobenzolgehalt im Produkt betrug 86 ppm.
Das Beispiel zeigt, dass der in Beispiel 1 bereits demonstrierte Austausch von Katalysator auch *ohne Unterbrechung des Hydrierprozesses* erfolgen kann, was für die großtechnische Anwendung einen enormen Vorteil darstellt. Der durchschnittliche Nitrobenzolgehalt im Produkt ist mit 86 ppm noch im akzeptablen Bereich.

**Beispiel 4 (Vergleichsbeispiel)**

Dieser Versuch wurde in einer dreistufigen adiabat betriebenen Festbett-Hydrieranlage mit Kreisgassystem durchgeführt. Die Reaktoren wurden mit jeweils ca. 540 ml Schüttvolumen Katalysator D, entsprechend einer Betthöhe von 20 cm, befüllt. Der Versuch wurde mit einer spezifischen Belastung im ersten Reaktor von 1,0 $g_{Nitrobenzol}$ / ($ml_{Kat.}$ · h) begonnen, welche, um bessere Selektivitäten zu erzielen, im Verlauf des Versuchs stufenweise erhöht wurde (nach 24 Std. auf 1,5 $g_{Nitrobenzol}$ / ($mi_{Kat.}$ · h) und nach 48 Std. auf 2,7 $g_{Nitrobenzol}$ / ($ml_{Kat.}$ · h)). Es wurde ein molares Verhältnis von Wasserstoff zu Nitrobenzol von ca. 80 : 1 eingesetzt; der absolute Druck betrug 4 bar, die Eingangstemperatur des Eduktgasgemisches etwa 240 °C. Die Fahrweise entspricht dem gängigen Stand der Technik bei adiabater Prozessführung. Der Versuch wurde 312 Std. betrieben; dann wurde Nitrobenzoldurchbruch in der Probennahmenstelle nach dem dritten Reaktor beobachtet. Bis dahin betrug der mittlere Gehalt an Nitrobenzol im Produkt nach dem ersten Reaktor 2 ppm.
Die Anlage erlaubt Probennahmen nach jedem Reaktor. Für den Vergleich mit Beispiel 3 wurden nur die Analysenresultate nach dem ersten Reaktor herangezogen.
Figur 4 stellt die in den Beispielen 3 und 4 erzielten durchschnittlichen Selektivitäten $S_D$ einander gegenüber. (Der Wert für $S_D(t)$ zu einem gegebenen Zeitpunkt t bezeichnet die gemittelte Selektivität, mit der das gesamte bis zu diesem Zeitpunkt angefallene Anilin hergestellt wurde.) Die Werte in Beispiel 4 sind diejenigen nach dem ersten Reaktor der dreistufigen Anlage.
In Beispiel 3 wurde das Anilin mit einer deutlich höheren Selektivität gebildet als im Vergleichsbeispiel 4. Obwohl in Beispiel 4 die spezifische Belastung erhöht wurde, um die Selektivität zu verbessern (Verringerung der Verweilzeit des gebildeten Anilins auf dem Katalysator), konnten nur deutlich schlechtere Resultate erzielt werden als im erfindungsgemäßen Beispiel. Die Selektivitätsverbesserung in Beispiel 3 gegenüber Beispiel 4 ist so groß, dass der Nachteil des höheren Nitrobenzolgehalts im Produkt überkompensiert wird. Die Wanderbettvariante aus Beispiel 3 erlaubt den Austausch von Katalysator ohne Unterbrechung des Hydrierprozesses, sodass die Aktivität des aktiv am Hydrierprozess beteiligten Katalysators nur innerhalb geringer Grenzen schwankt, und zwar auf einem solchen Niveau, dass daraus von Beginn an hohe Selektivitäten resultieren.

**Patentansprüche**

1. Verfahren zur Herstellung von aromatischen Aminen der Formel

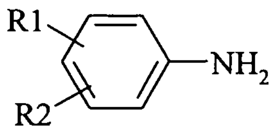

(I)

in der R1 und R2 unabhängig voneinander Wasserstoff, Methyl oder Ethyl bedeuten, wobei R1 zusätzlich Amino bedeuten kann, durch Hydrierung von Nitroaromaten der Formel

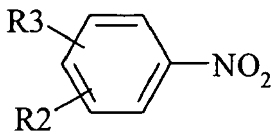

(II)

in der R2 und R3 unabhängig voneinander Wasserstoff, Methyl oder Ethyl bedeuten, wobei R3 zusätzlich Nitro bedeuten kann,
in der Gasphase mit Wasserstoff an in stationären oder quasi-stationären Betten angeordneten Katalysatoren in einem Reaktor, **dadurch gekennzeichnet, dass** der in dem Reaktor befindliche Katalysator kontinuierlich oder in

periodischen Abständen zumindest teilweise ausgetauscht wird, wobei
bei kontinuierlichem Katalysatoraustausch innerhalb von Zeitspannen von jeweils 20 Tagen ab Beginn der Hydrierung
und
bei Katalysatoraustausch in periodischen Abständen innerhalb von 20 Tagen nach Beginn eines jeden Betriebszeitraums, wobei ein Betriebzeitraum die Zeitspanne zwischen zwei zumindest teilweise durchgeführten Katalysatoraustausch-Vorgängen, inklusive der Erstbefüllung des Reaktors, ist,
mindestens 10 % des Katalysators ausgetauscht werden.

**2.** Verfahren nach Anspruch 1, **dadurch gekennzeichnet, dass** der Katalysator im Reaktor in Form eines oder mehrerer quasi-stationärer Katalysatorbetten angeordnet ist,

(i) die Katalysatorbetten in Form von einer oder mehreren regelmäßig geformten flächigen Katalysatorschichten im Reaktor angeordnet sind,
(ii) die Entnahme von Katalysator aus einer flächigen Katalysatorschicht und die Zuführung von Katalysator in eine flächige Katalysatorschicht ohne Unterbrechung des Hydrierprozesses kontinuierlich oder in periodischen Abständen erfolgen,
(iii) die flächige Katalysatorschicht bzw. die erste von mehreren in Reihe geschalteten flächigen Katalysatorschichten mit einem Gasgemisch angeströmt wird, das pro Mol Nitrogruppe 3 Mol bis 150 Mol Wasserstoff enthält,
(iv) die Hydrierung bei einem absoluten Druck von 1 bar bis 50 bar, bei einer Eingangstemperatur des eingesetzten Gasgemisches von 150 °C bis 400 °C und einer maximalen Katalysatortemperatur von 600 °C unter adiabaten Bedingungen durchgeführt wird,
(v) aus dem bei der Hydrierung erhaltenen Reaktionsgemisch Wasserstoff abgetrennt und der so erhaltene Wasserstoff in die Hydrierung zurückgeführt wird.

**3.** Verfahren nach Anspruch 2, **dadurch gekennzeichnet, dass** die Strömungsrichtung des Gasgemisches im Wesentlichen senkrecht zur Richtung des Katalysatoraustrags erfolgt und dass die Länge $L_E$ der flächigen Katalysatorschichten in Richtung des Eduktgasstromes kleiner ist als die Länge $L_K$ der flächigen Katalysatorschichten in Richtung des Katalysatoraustrags, wobei $L_E$ zwischen 1 cm und kleiner 100 cm und $L_K$ maximal 20 m betragen.

**4.** Verfahren nach einem der Ansprüche 2 bis 3, **dadurch gekennzeichnet, dass** die flächige Katalysatorschicht bzw. die erste von mehreren in Reihe geschalteten flächigen Katalysatorschichten mit einem Gasgemisch angeströmt wird, das pro Mol Nitrogruppe 3 Mol bis 150 Mol Wasserstoff *und* 0,01 Mol bis 100 Mol Wasser enthält.

**5.** Verfahren nach einem der Ansprüche 2 bis 4, **dadurch gekennzeichnet, dass** der dem Reaktor entnommene Katalysator
in Massenanteilen von 1 % bis 100 %, bezogen auf die Gesamtmasse des in einem Zeitintervall entnommenen Katalysators, in die Hydrierung zurückgeführt wird und
vor der Rückführung in die Hydrierung in Massenanteilen von 0,1 % bis 100 %, bezogen auf die Gesamtmasse des in die Hydrierung zurückzuführenden Katalysators, mit sauerstoffhaltigen Gasgemischen bei Temperaturen zwischen 100 °C und 400 °C regeneriert wird und
die nicht regenerierten Anteile zusammen mit den regenerierten Anteilen in die Hydrierung zurückgeführt werden.

**6.** Verfahren nach Anspruch 5, **dadurch gekennzeichnet, dass** die Regeneration nur unvollständig durchgeführt wird.

**7.** Verfahren nach Anspruch 5, **dadurch gekennzeichnet, dass** Katalysator vor oder nach Rückführung in den Reaktor homogenisiert wird.

**8.** Verfahren nach einem der Ansprüche 2 bis 7, **dadurch gekennzeichnet, dass** Katalysatorabrieb laufend oder periodisch aus dem Verfahren ausgetragen und durch Zufuhr von Katalysator von außen ersetzt wird.

**9.** Verfahren nach Anspruch 1, **dadurch gekennzeichnet, dass** als Nitroaromat Nitrobenzol oder Nitrotoluol eingesetzt werden.

**10.** Verfahren nach Anspruch 1, **dadurch gekennzeichnet, dass** Katalysatoren eingesetzt werden, die Palladium in Gehalten von 1 - 100 $g/l_{Katalysator}$ und Vanadium in Gehalten von 0,01 - 100 $g/l_{Katalysator}$ oder
Palladium in Gehalten von 1 - 100 $g/l_{Katalystor}$ und Vanadium in Gehalten von 0,01 - 100 $g/l_{Katalysator}$ und Blei in

Gehalten von 0,01 - 100 $g/l_{Katalysator}$
oder
Palladium in Gehalten von 1 - 100 $g/l_{Katalystor}$ und Vanadium in Gehalten von 0,01 - 100 $g/l_{Katalysator}$ und Gallium in Gehalten von 0,01 - 100 $g/l_{Katalysator}$
auf einem Träger aus $\alpha$-Aluminiumoxid mit einer BET-Oberfläche von weniger als 50 $m^2/g$ und einer Bruchkraft von größer als 30 N enthalten.

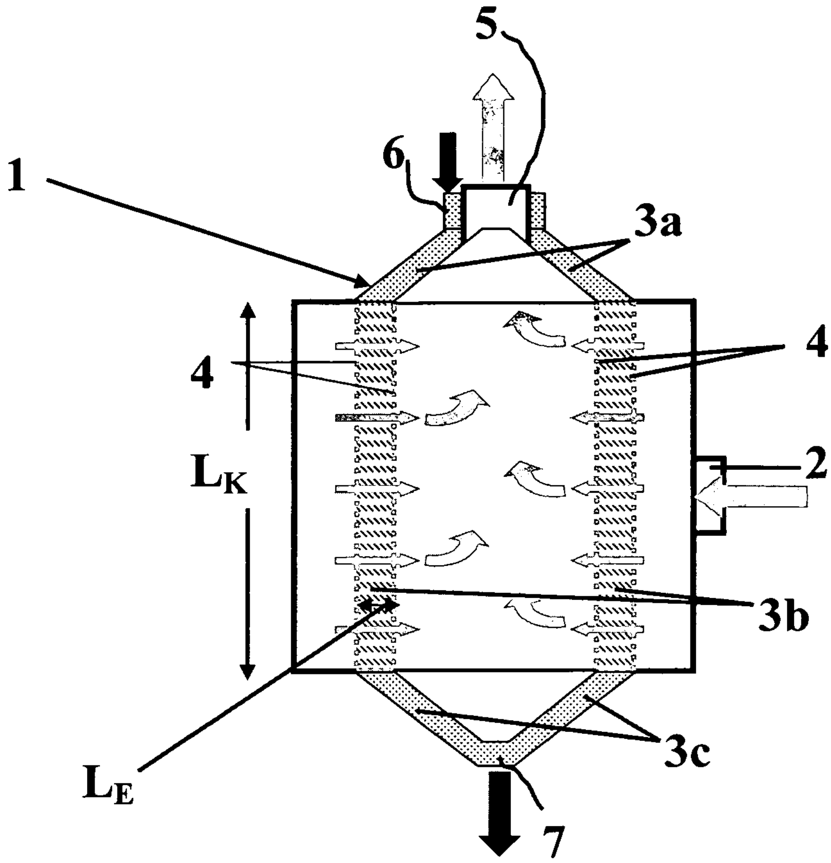


Fig. 1a

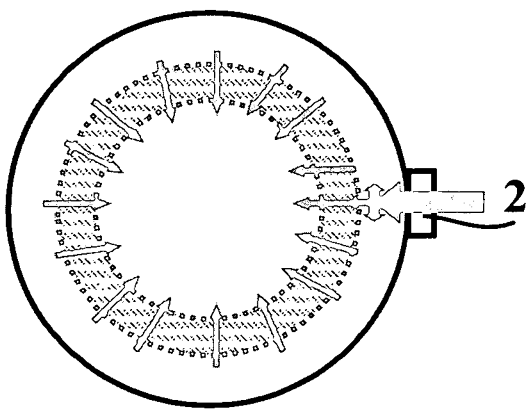


Fig. 1b

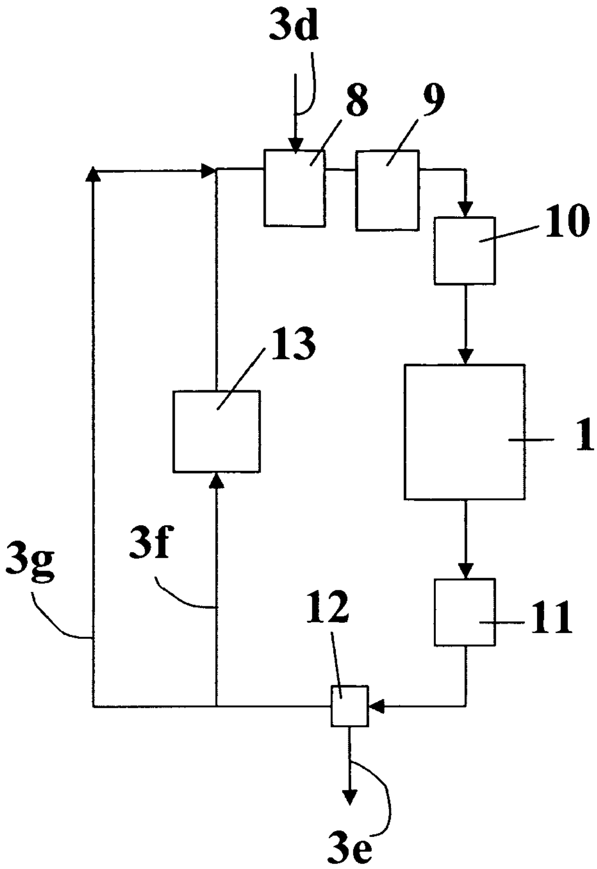


Fig. 2

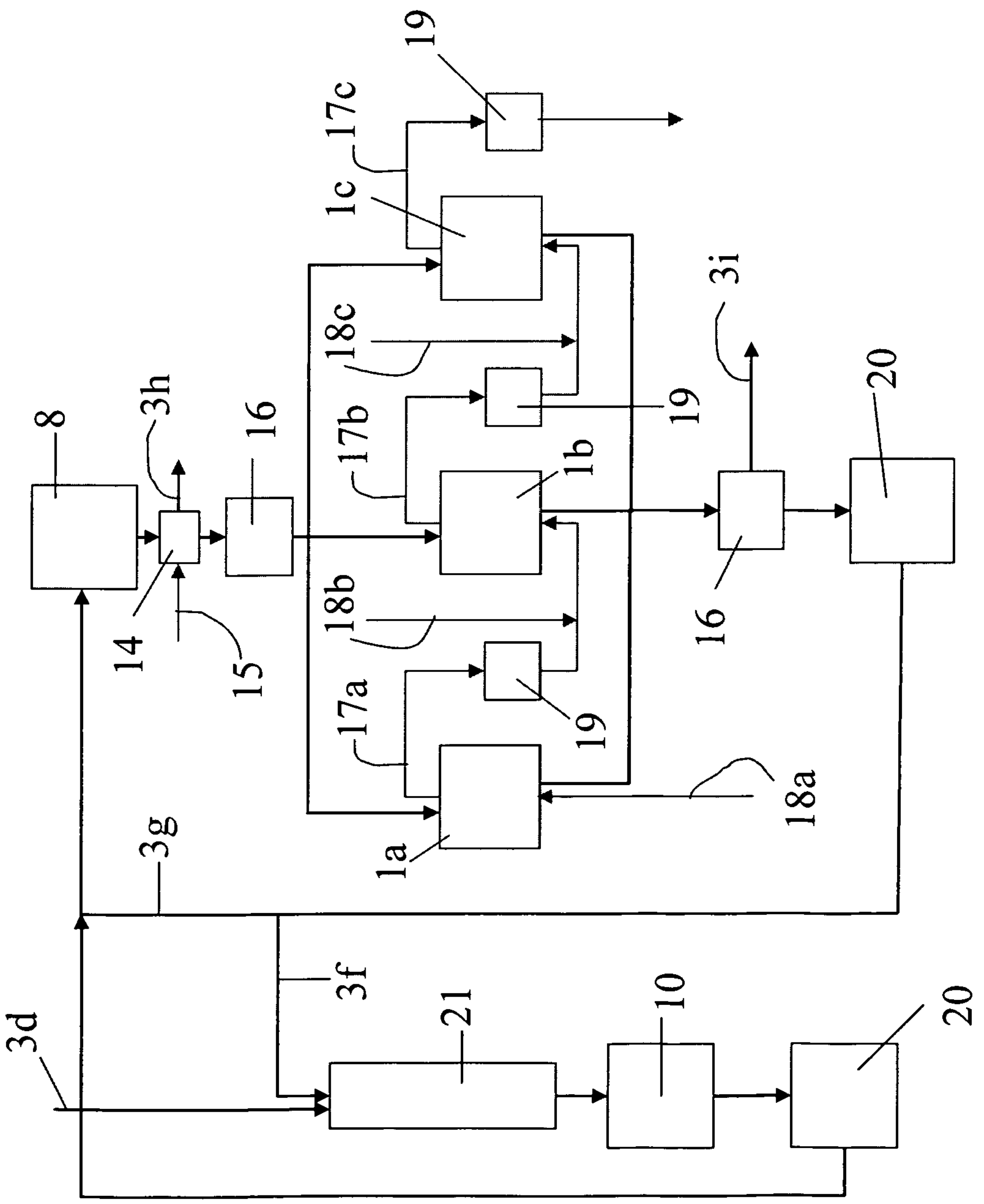
8
3h
14
15
16
17a
17b
17c
18a
18b
18c
19
19
19
1a
1b
1c
3i
16
20
3g
3f
3d
21
10
20

Figur 3

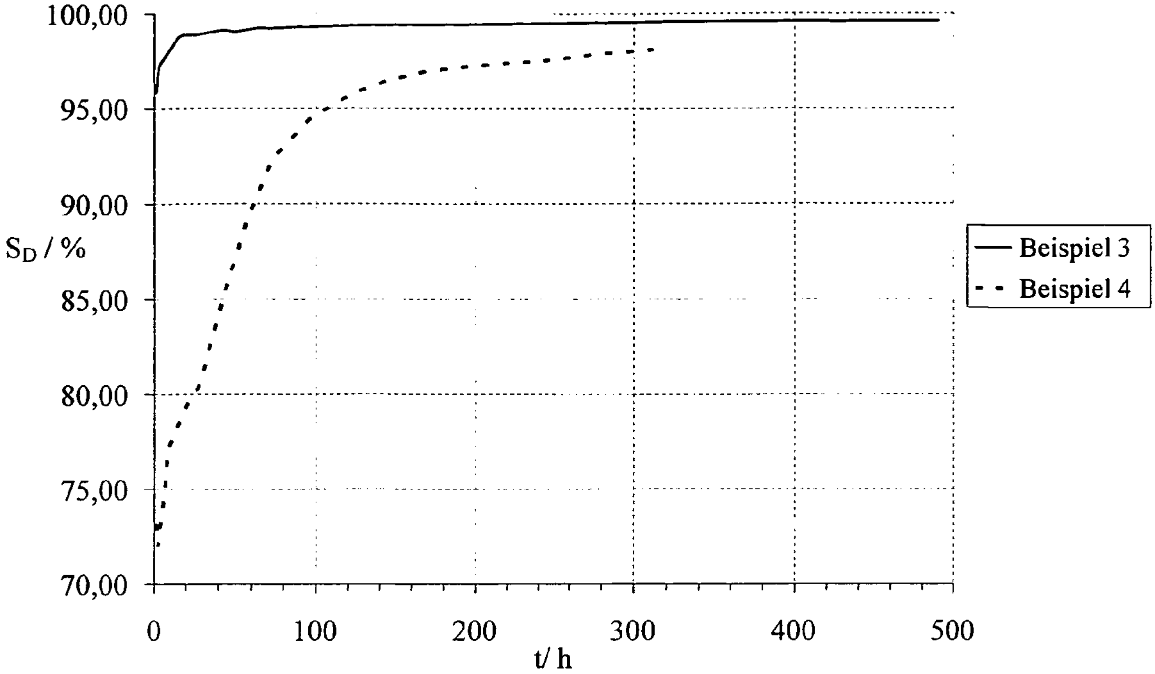
100,00
95,00
90,00
85,00
80,00
75,00
70,00
$S_D$ / %
0
100
200
300
400
500
t/ h
Beispiel 3
Beispiel 4

Fig. 4

Europäisches Patentamt
European Patent Office
Office européen des brevets

# EUROPÄISCHER RECHERCHENBERICHT

Nummer der Anmeldung
EP 10 00 4248

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 0 716 061 A1 (BASF AG [DE]) 12. Juni 1996 (1996-06-12)<br>* Seite 2, Zeile 43 - Seite 3, Zeile 59 *<br>* Seite 2, Zeile 48: "räumlichen Wechsel" *<br>* Seite 3, Zeile 9: "Wanderbett" *<br>* Seite 2, Zeile 10 *<br>* Seite 5, Zeile 51 - Zeile 59 *<br>* Seite 6, Zeile 39 - Zeile 50 *<br>----- | 1-10 | INV.<br>C07C209/36<br>C07C211/46 |
| A,D | DE 22 44 401 A1 (BAYER AG) 16. Mai 1974 (1974-05-16)<br>* Seite 12, Tablelle, die vier letzten Einträge *<br>----- | 1 | |
| A,D | US 4 188 283 A (CZAJKOWSKI GEORGE J [US] ET AL) 12. Februar 1980 (1980-02-12)<br>* Zusammenfassung *<br>----- | 1 | |
| | | | RECHERCHIERTE SACHGEBIETE (IPC) |
| | | | C07C |

Der vorliegende Recherchenbericht wurde für alle Patentansprüche erstellt

1

| Recherchenort | Abschlußdatum der Recherche | Prüfer |
|---|---|---|
| Den Haag | 10. Juni 2010 | Fitz, Wolfgang |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 10 00 4248

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-06-2010

| Im Recherchenbericht angeführtes Patentdokument | Datum der Veröffentlichung | Mitglied(er) der Patentfamilie | Datum der Veröffentlichung |
|---|---|---|---|
| EP 0716061 A1 | 12-06-1996 | DE 4443360 A1 | 13-06-1996 |
| | | JP 8225468 A | 03-09-1996 |
| | | US 5689005 A | 18-11-1997 |
| DE 2244401 A1 | 16-05-1974 | KEINE | |
| US 4188283 A | 12-02-1980 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 1114820 B
- DE 1133394 B
- WO 2008034770 A1
- DE OS2201528 A
- GB 1452466 A
- DE OS1809711 A
- DE OS3636984 A
- DE 2244401 A
- DE 2849002 A
- DE 4039026 A1
- DE 19651688 A1
- EP 0696573 B1
- EP 0696574 B1
- EP 0748789 B1
- EP 0748790 B1
- EP 1882681 A1
- EP 0696574 A
- DE 4207905 A1
- CN 101016247 A
- US 1982099 A
- US 1995293 A
- US 3647680 A
- US 4133743 A
- US 4188283 A
- CN 1454970 A
- CN 1333084 A
- US 20060063957 A
- US 3706536 A
- EP 0154492 A
- US 20060122446 A1
- US 20060115387 A1
- DE OS2849002 A